(19) Europäisches Patentamt
European Patent Office
Office européen des brevets

(11) Veröffentlichungsnummer: **0 170 861**
**B1**

(12) # EUROPÄISCHE PATENTSCHRIFT

(45) Veröffentlichungstag der Patentschrift: **09.03.88**

(51) Int. Cl.⁴: **C 07 D 231/20**, C 07 D 405/04, A 61 K 31/415

(21) Anmeldenummer: **85107960.8**

(22) Anmeldetag: **27.06.85**

(54) **3-Aminocarbonylmethoxy-5-phenylpyrazol-Verbindungen sowie Verfahren zu ihrer Herstellung und diese Verbindungen enthaltende Arzneimittel.**

(30) Priorität: **04.07.84 DE 3424586**

(43) Veröffentlichungstag der Anmeldung: **12.02.86 Patentblatt 86/7**

(45) Bekanntmachung des Hinweises auf die Patenterteilung: **09.03.88 Patentblatt 88/10**

(84) Benannte Vertragsstaaten: **AT BE CH DE FR GB IT LI LU NL SE**

(56) Entgegenhaltungen:
**EP - A - 0 007 019**
**DE - A - 2 809 183**

(73) Patentinhaber: **Kali-Chemie Pharma GmbH, Hans-Böckler-Allee 20 Postfach 220, D-3000 Hannover 1 (DE)**

(72) Erfinder: **Heinemann, Henning, Dipl.-Chem. Dr. rer. nat., Bergiusstrasse 18, D-3000 Hannover 51 (DE)**
Erfinder: **Kehrbach, Wolfgang, Dipl.-Chem. Dr. rer. nat., Altenbekener Damm 41, D-3000 Hannover 1 (DE)**
Erfinder: **Schön, Uwe, Dr. Dipl.-Chem., Föhrenkamp 12, 3167 Burgdorf (DE)**
Erfinder: **Buschmann, Gerd, Dr. med. vet., Ernst-Ebeling-Strasse 9, D-3000 Hannover 72 (DE)**
Erfinder: **Kühl, Ulrich, Dr. med. vet., Franzburger Strasse 10, D-3007 Gehrden 1 (DE)**

(74) Vertreter: **Lauer, Dieter, Dr., c/o Kali-Chemie AG Postfach 220 Hans-Böckler-Allee 20, D-3000 Hannover 1 (DE)**

EP 0 170 861 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue 3-(Aminoalkylaminocarbonylmethoxy)-5-phenylpyrazol-Verbindungen und deren Salze sowie diese Verbindungen enthaltende pharmazeutische Zubereitungen und Verfahren zur Herstellung dieser Verbindungen.

Aus der europäischen Patentanmeldung Nr. 7019 sind 3-Hydroxycarbonylmethoxy-5-phenylpyrazol-Verbindungen und deren Ester und Amide mit blutlipidsenkenden Eigenschaften bekannt.

Der vorliegenden Erfindung liegt die Aufgabe zugrunde, neue 3-Carbonylmethoxy-5-phenylpyrazol-Verbindungen mit wertvollen pharmakologischen Eigenschaften zu entwickeln.

Es wurde nun gefunden, dass die neuen in 3-Stellung durch einen Aminoalkylaminocarbonylmethoxyrest substituierten 5-Phenylpyrazol-Verbindungen wertvolle pharmakologische Eigenschaften besitzen und sich insbesondere durch ausgeprägte antiarrhythmische Wirkungen auszeichnen und ein vorteilhaftes Wirkungsprofil aufweisen. Aufgrund ihrer pharmakologischen Wirkungen sind die neuen Verbindungen als Arzneimittel, insbesondere zu Behandlung und Prophylaxe von Herzrhythmusstörungen, geeignet.

Die vorliegende Erfindung betrifft dahe neue 3-Aminocarbonylmethoxy-5-phenylpyrazol-Verbindungen der allgemeinen Formel I

(siehe Formel I)

worin

$R_1$ Wasserstoff oder Alkyl mit 1-4 Kohlenstoffatomen bedeutet

$R_2$ in 1- oder 2-Stellung angeordnet ist und Wasserstoff oder Alkyl mit 1-4 Kohlenstoffatomen bedeutet

$R_3$ Wasserstoff, Halogen, Alkyl mit 1-4 Kohlenstoffatomen, Alkoxy mit 1-4 Kohlenstoffatomen bedeutet und

$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder

$R_3$ und $R_4$ an zwei benachbarte Kohlenstoffatome gebunden sind und gemeinsam eine Alkylendioxygruppe mit 1-2 Kohlenstoffatomen darstellen,

$R_5$ Wasserstoff oder Alkyl mit 1-4 Kohlenstoffatomen bedeutet und

$R_6$ Wasserstoff oder Methyl bedeutet oder

$R_5$ und $R_6$ gemeinsam eine Alkylenkette mit 3-5 Kohlenstoffatomen bilden,

$R_7$ Wasserstoff oder Alkyl mit 1-4 Kohlenstoffatomen bedeutet,

Z eine Alkylenkette mit 2-5 Kohlenstoffatomen oder die 2-Hydroxypropylenkette bedeutet,

$R_8$ Wasserstoff oder Alkyl mit 1-4 Kohlenstoffatomen bedeutet und

$R_9$ Wasserstoff oder Alkyl mit 1-4 Kohlenstoffatomen bedeutet oder

$R_8$ und $R_9$ gemeinsam mit dem Stickstoffatom, an das sie gebunden sind, eine heterocyclische Gruppe der allgemeinen Formel a

(siehe Formel a)

darstellen, worin A für eine gegebenenfalls durch 1-2 Methylgruppen substituierte Alkylenkette mit 4-5 Kohlenstoffatomen oder die $C_2H_4$-O-$C_2H_4$-kette steht, oder falls Z eine Alkylenkette ist, auch

$R_8$ Wasserstoff oder Alkyl mit 1-4 Kohlenstoffatomen bedeutet und

$R_9$ eine Alkylenkette ist und zusammen mit dem Stickstoffatom, an welches es gebunden ist, und dem diesem Stickstoffatom benachbarten Kohlenstoffatom der Alkylenkette Z einen Pyrrolidin- oder Piperidinring bildet,

sowie deren Säureadditionssalze.

Sofern in den Verbindungen der Formel I die Substituenten $R_1$ bis $R_2$ niedere Alkylgruppen darstellen oder enthalten, stellen diese gerade oder verzweigte Alkylgruppen mit vorzugsweise 1-4 Kohlenstoffatomen, insbesondere Methyl oder Äthyl dar.

$R_1$ stellt vorzugsweise Wasserstoff dar. Falls $R_1$ eine niedere Aklylgruppe ist, handelt es sich vorzugsweise um Methyl oder Äthyl. $R_2$ stellt vorzugsweise Wasserstoff dar. Falls $R_2$ niederes Aklyl bedeutet, stellt dieses bevorzugt Methyl dar.

In den Substituenten $R_3$ und $R_4$ des Phenylringes stellen niedere Alkyl- oder Alkoxygruppen bevorzugt Methyl oder Methoxy dar. Als Halogenatome kommen insbesondere Fluor, Chlor oder Brom, bevorzugt Fluor in Frage. Vorzugsweise stellen $R_3$ und/oder $R_4$ Wasserstoff oder auch Fluor, Chlor oder Methyl dar.

$R_5$ stellt vorzugsweise eine bevorzugt primäre niedere Alkylgruppe mit 1-4 insbesondere 1-2 Kohlenstoffatomen dar, welche bevorzugt geradkettig ist. Vorzugsweise sind $R_5$ und $R_6$ je Methyl. Falls $R_5$ mindestens 2 Kohlenstoffatome enthält, ist $R_6$ bevorzugt Wasserstoff. Falls $R_5$ und $R_6$ gemeinsam eine Alkylenkette bilden, stellt diese vorzugsweise Propylen dar. Vorteilhafterweise enthalten $R_5$ und $R_6$ zusammen 2-3 Kohlenstoffatome, $R_7$ stellt bevorzugt Wasserstoff dar. Falls $R_7$ eine Alkylgruppe bedeutet, stellt diese eine bevorzugt primäre Alkylgruppe mit 1-4, insbesondere 1-2 Kohlenstoffatomen dar.

Falls Z eine Alkylenkette darstellt ist dies vorzugsweise eine gerade Kette mit 2-4 Kohlenstoffatomen.

Falls $R_8$ und/oder $R_9$ niedere Alkylgruppen bedeuten, können diese gerade oder verzweigt sein und 1-4, vorzugsweise 1-2 Kohlenstoffatome enthalten. Zweckmässigerweise stellt mindestens einer der Substituenten $R_8$ und $R_9$ eine nieder Alkylgruppe dar oder ist Teil eines heterocyclischen Ringes. Vorteilhaft stellt die $NR_8R_9$-Gruppe eine vorzugsweise unverzweigte Dialkylaminogruppe, insbesondere die Diäthylaminogruppe dar. Als Beispiele von aus dem Rest $R_9$ und dem Stickstoffatom, an welches er gebunden ist, zusammen mit dem Rest $R_8$ oder dem dem Stickstoff benachbarten C-Atom der Alkylenkette Z gebildeten heterocyclischen Ringen seien genannt Piperidin, Morpholin oder Pyrrolidine.

Die neuen 3-Aminocarbonylmethoxy-5-phenylpyrazol-Verbindungen der Formel I und deren Säureadditionssalze werden erfindungsgemäss erhalten, in dem man in an sich bekannter Weise

a) Verbindungen der allgemeinen Formel II oder III

(siehe Formel II und III)

worin $R_1$, $R_2$, $R_3$, $R_4$, $R_5$ und $R_6$ obige Bedeutung besitzen und Y eine reaktive Gruppe bedeutet, mit Verbindungen der allgemeinen Formel VIII

(siehe Formel VIII)

worin $R_7$, Z, $R_8$ und $R_9$ obige Bedeutungen besitzen, umsetzt, oder

b) Verbindungen der allgemeinen Formel IV

(siehe Formel IV)

worin $R_1$, $R_2$, $R_3$, $R_4$, $R_5$, $R_6$, $R_7$ und Z obige Bedeutung besitzen und X eine aminolytisch abspaltbare Gruppe bedeutet, mit Verbindungen der allgemeinen Formel V

(siehe Formel V)

worin $R_8$ und $R_9$ obige Bedeutung besitzen, umsetzt, oder

c) Verbindungen der allgemeinen Formel VI

(siehe Formel VI)

worin $R_1$, $R_2$, $R_3$ und $R_4$ obige Bedeutung besitzen, und/oder die dazu tautomeren 5-Phenylpyrazolin-3-on-Verbindungen mit Verbindungen der allgemeinen Formel VII

(siehe Formel VII)

worin $R_5$, $R_6$, $R_7$, Z, $R_8$ und $R_9$ obige Bedeutung besitzen und Hal Halogen bedeutet, umsetzt, oder

d) zur Herstellung von Verbindungen der allgemeinen Formel Ia

(siehe Formel Ia)

worin $R_1$, $R_2$, $R_3$, $R_4$, $R_5$, $R_6$, $R_7$, $R_8$ und $R_9$ obige Bedeutung besitzen, Verbindungen der allgemeinen Formel IX

(siehe Formel IX)

worin $R_1$, $R_2$, $R_3$, $R_4$, $R_5$, $R_6$ und $R_7$ obige Bedeutung besitzen, umsetzt mit einer Verbindung der allgemeinen Formel V

(siehe Formel V)

und gegebenenfalls in Verbindungen der allgemeinen Formel I, worin $R_4$ Methoxy bedeutet, die Methoxygruppe zur Hydroxygruppe spaltet,
und gegebenenfalls freie Verbindungen der Formel I in ihre Säureadditionssalze überführt oder die Säureadditionssalze in die freien Verbindungen der Formel I überführt.

Für die Umsetzung gemäss Verfahrensvariante a) können die Säuren oder Säurederivate der Formel II oder, sofern es sich um Verbindungen handelt, worin $R_2$ Wasserstoff ist, auch die cyclisierten Säurederivate der Formel III oder Gemische aus Verbindungen der Formeln II und III eingesetzt werden.

Die Umsetzung der Säuren oder Säurederivate der Formeln II oder III mit Diaminen der Formel VIII kann nach an sich zur Bildung von Amid-Gruppierungen durch Aminoacylierung üblichen Methoden ausgeführt werden. Es können die Säuren der Formel II (Y = OH) oder deren reaktionsfähige Derivate, worin Y eine reaktionsfähige Gruppe bedeutet, oder deren cyclische Derivate der Formel III eingesetzt werden. Als reaktionsfähige Derivate der Formel II kommen insbesondere Säurehalogenide, vorzugsweise -chloride, Ester und gemische Anhydride in Frage, z.B. Verbindungen der Formel II, worin die reaktive Gruppe Y Halogen, insbesondere Chlor oder Brom, niederes Alkoxy, insbesondere Alkoxy mit 1-4 Kohlenstoffatomen, oder eine Gruppe O-W bedeutet, worin W für eine Niederalkylcarbonyl- oder Niederalkoxycarbonyl-Gruppe oder einen organischen Sulfonsäurerest, insbesondere den Rest einer Niederalkansulfonsäure wie z.B. Methansulfonsäure oder einer aromatischen Sulfonsäure wie Benzolsulfonsäure oder durch niederes Alkyl oder Halogen substituierte Benzolsulfonsäuren, steht.

Falls eine Säure der Formel II selbst eingesetzt wird, so wird die Umsetzung zweckmässigerweise in Gegenwart eines als zur Amidbildung geeignet bekannten Kopplungsreagenzes durchgeführt. Geeignete Kopplungsreagenzien, welche die Amidbildung dadurch fördern, dass sie mit der Säure in situ unter Bildung eines reaktionsfähigen Säurederivates reagieren sind aus der Peptidchemie bekannt. Als Beispiele geeigneter Kopplungsreagenzien seien genannt Alkyl-, vorzugsweise Cycloalkylcarbodiimide wie Dicyclohexylcarbodiimid, Carbonyldiimidazol und N-Niederalkyl-2-halogenpyridiniumsalze, insbesondere Halogenide oder Tosylate, vorzugsweise N-Methyl-2-chlorpyridiniumjodid (siehe z.B. Mukaiyama in Angew. Chemie 91, Seite 789 bis 812). Die Umsetzung in Gegenwart eines Kopplungsreagenzes kann zweckmässig bei Temperaturen von −30°C bis Raumtemperatur unter Benutzung von Lösungsmitteln wie halogenierten Kohlenwasserstoffen und/oder aromatischen Lösungsmitteln gegebenenfalls in Gegenwart eines säurebindenden Amins durchgeführt werden.

Vorzugsweise werden als Verbindungen der Formel II Ester oder auch Säurehalogenide, insbesondere Säurechloride, oder gemischte Säureanhydride, insbesondere aus Umsetzung der Säuren der Formel II mit einem organischen Sulfonsäurechlorid wie Methansulfonsäurechlorid, oder mit Chlorameisensäureester erhaltene gemischte Anhydride, oder auch cyclische Derivate der Formel III verwendet. Die Umsetzung des Amins mit dem Säurehalogenid oder -anhydrid und/oder mit einem cyclischen Derivat der Formel III wird in Gegenwart eines inerten organischen Lösungsmittels beispielsweise eines halogenierten Kohlenwasserstoffes wie Methylenchlorid, eins cyclischen oder offenen Äthers wie Dioxan oder Diäthyläther, Dimethylformamid, Sulfolan, Tetramethylharnstoff oder Gemischen dieser Lösungsmittel und gegebenenfalls aromatischer Kohlenwasserstoffe wie Benzol oder Toluol durchgeführt. Sofern

Säurehalogenide oder -anhydride der Formel II eingesetzt werden, ist es zweckmässig, die Reaktion in Gegenwart eines säurebindenden Mittels durchzuführen. Als säurebindende Mittel eignen sich anorganische Basen, beispielsweise Alkalimetallcarbonate oder -hydroxide oder organische Basen, insbesondere tertiäre Niederalkylamine, z.B. Triäthylamin oder Pyridine. Statt einer Fremdbase kann auch ein Überschuss des Amins der Formel VIII verwendet werden. Im Überschuss eingesetzte organische Basen können gleichzeitig auch als Lösungsmittel dienen. Es kann vorteilhaft sein darüber hinaus katalytische Mengen von basischen Pyridinen wie 4-Dimethylaminopyridin oder 4-Pyrrolidinopyridin zuzusetzen. Vorteilhafterweise wird die Reaktion bei Temperaturen zwischen −30°C und Siedetemperatur des Reaktionsgemisches durchgeführt. Die gewählte Temperatur kann je nach den eingesetzten Ausgangsverbindungen variieren, beispielsweise sind bei Verwendung von Säurehalogeniden oder -anhydriden der Formel II und cyclischen Derivaten der Formel III niedrige Temperaturen bis ca. Raumtemperatur bevorzugt.

Insbesondere erweist es sich als vorteilhaft, eine stark gekühlte Lösung des Säurederivates der Formel II oder III mit einer Lösung des Amins der Formel VIII umzusetzen. Falls das Amin eine zweite reaktive Aminfunktion enthält, ist es zweckmässig die Lösung des Säurederivates allmählich zu der Aminlösung zu geben.

Wenn als Ausgangsverbindungen Verbindungen der Formel II eingesetzt werden, worin $R_2$ Wasserstoff ist, können diese unter den vorgenannten Reaktionsbedingungen, zunächst teilweise in die entsprechenden cyclischen Verbindungen der Formel II umgewandelt werden, welche dann mit den Aminen der Formel VIII weiter reagieren.

Die Umsetzung von Verbindungen der Formel IV mit Aminen der Formel V gemäss Verfahrensvariante b) kann nach an sich zu Aminoalkylierung üblichen Methoden durchgeführt werden. Als aminolytisch abspaltbare Reste in den Verbindungen der Formel IV kommen bevorzugt Halogene wie Chlor, Brom, Jod oder auch organische Sulfonsäurereste in Frage, insbesonders Reste von Niederalkylsulfonsäuren wie z.B. Methansulfonsäure oder von aromatischen Sulfonsäuren, wie Benzolsulfonsäure oder durch niederes Alkyl oder Halogen substituierten Benzolsulfonsäuren z.B. Toluolsulfonsäuren oder Brombenzolsulfonsäuren. Zweckmäsigerweise wird die Reaktion in einem unter den Reaktionsbedingungen inerten organischen Lösungsmittel durchgeführt.

Als Beispiele geeigneter Lösungsmittel seien genannt aromatische Kohlenwasserstoffe wie Benzol, Toluol oder Xylol, cyclische Äther wie Dioxan, Dimethylformamid, Sulfolan, Tetramethylharnstoff oder Dimethylsulfoxid. Zweckmässigerweise wird ein Überschuss des Amins der Formel V als säurebindendes Mittel eingesetzt. Ein Überschuss des Amins kann auch als Lösungsmittel dienen. Gewünschtenfalls können auch weitere anorganische Basen als säurebindende Mittel zugesetzt werden. Geeignete anorganische Basen sind beispielsweise Alkalimetallcarbonate oder -bicarbonate.

Die Umsetzung gemäss Verfahrensvariante c) stellt eine an sich bekannte Alkylierung am Sauerstoffatom in 3-Stellung des 3-Hydroxypyrazols bzw. des dazu tautomeren Pyrazolin-3-ons dar. Die Umsetzung erfolgt zweckmässigerweise unter basischen Bedingungen in einem inerten Lösungsmittel beispielsweise Dimethylformamid, cyclischen Äthern wie Dioxan, Tetramethylharnstoff, Dimethylsulfoxid, aromatischen Kohlenwasserstoffen wie Toluol oder deren Gemischen. Als Basen eignen sich insbesondere anorganische Basen wie Alkalimetallcarbonate oder -hydroxyde. Zweckmässigerweise werden ca. äquivalente Mengen der Verbindung der Formel VI und der anorganischen Base eingesetzt. Gewünschtenfalls können die Verbindungen der Formel VII auch umgesetzt werden mit Alkalimetallsalzen der Verbindungen der Formel VI welche in situ durch Umsetzung der Verbindungen der Formel VI in einen der vorgenannten Lösungsmittel mit einem Alkalimetallhydrid hergestellt werden können. Die Umsetzung erfolgt vorzugsweise bei Temperaturen zwischen 0°C und Siedetemperatur des Lösungsmittels, vorzugsweise bei Temperaturen zwischen ca. 0 und 100°C.

Die Umsetzung von Verbindungen der Formel IX mit Verbindungen der Formel V gemäss Verfahrensvariante d) kann auf an sich zu Umsetzung von Epoxiden übliche Weise erfolgen. Zweckmässigerweise wird die Umsetzung in einem unter den Reaktionsbedingungen inerten organischen Lösungsmittel bei Temperaturen zwischen ca. 0°C und ca. 100°C durchgeführt. Als Lösungsmittel eignen sich beispielsweise aromatische Kohlenwasserstoffe wie Benzol, Toluol oder Xylol, offene oder cyclische Äther wie z.B. Diäthyläther, Tetrahydrofuran oder Dioxan oder niedere Alkohole.

In Verbindungen der Formel I, worin $R_4$ Methoxy bedeutet, kann die Methoxygruppe mit zur Spaltung von Methoxyaryläthern geeigneten Methoden auf an sich bekannte Weise zur Hydroxygruppe gespalten werden. Beispielsweise kann die Ätherspaltung durch Behandeln mit Jodwasserstoff in Acetanhydrid oder mit Lithiumjodid in Pyridinen, z.B. 2,4,6-Trimethylpyridin, erfolgen.

Falls die Ausgangsverbindungen freie Hydroxygruppen enthalten, können diese gewünschtenfalls vor den obigen Umsetzungen in an sich bekannter Weise mit einer Schutzgruppe versehen werden, welche anschliessend leicht wieder abspaltbar ist. Geeignete Schutzgruppen sind. z.B. bekannt aus E. McOmie «Protective Groups in Organic Chemistry» Plenum Press 1971. Beispielsweise eignen sich zum Schutz einer Hydroxygruppe hydrolytisch oder hydrogenolytisch spaltbare Äther wie Tetrahydropyranyläther oder Benzyläther.

Die Verbindungen der Formel 1 können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Als pharmakologisch annehmbare Säureadditionssalze der Verbindungen der Formel I eignen sich beispielsweise deren Salze mit anorganischen Säuren wie Chlorwasserstoffsäure, Schwefelsäure, Phosphorsäure oder organischen Säuren wie Methansulfonsäure, Äthansulfonsäure,

Cyclohexylaminosulfonsäure, Aminosulfonsäure, Essigsäure, Maleinsäure, Fumarsäure, Milchsäure, Weinsäure, Phenylessigsäure oder Mandelsäure.

Die Ausgangsverbindungen der Formel II und VI sind bekannt oder können nach an sich bekannten Verfahren hergestellt werden.

Es ist bekannt, dass die Verbindungen der Formel VI in mehreren tautomeren Formen vorliegen und dass neben der durch die Formel VI wiedergegebenen Enolform der 5-Phenylpyrazol-3-ole auch die entsprechende Ketoform der 5-Phenylpyrazolin-3-one existiert. Im allgemeinen liegen Gemische aus den verschiedenen tautomeren Formen vor, deren Zusammensetzung je nach Art der Substituenten variieren kann. Beide Formen, bzw. deren Gemische, können zur erfindungsgemässen Herstellung von Verbindungen der Formel I verwendet werden. Mit Verbindungen der Formel VI sind daher in der vorliegenden Anmeldung alle tautomeren Formen dieser Verbindungen gemeint.

Verbindungen der Formel VI können auf an sich zur Herstellung von 5-Phenylpyrazolin-3-onen bekannten Verfahren erhalten werden durch cyclisierende Kondensation von gegebenenfalls substituenten Hydrazinen der Formel XII

(siehe Formel XII)

worin $R_2$ obige Bedeutung besitzt, mit Benzoylessigsäureestern der Formel XIII

(siehe Formel XIII)

worin $R_1$, $R_3$ und $R_4$ obige Bedeutung besitzen, oder mit Phenylpropiolsäureestern der Formel XIV

(siehe Formel XIV)

worin $R_3$ und $R_4$ obige Bedeutung besitzen. Die Umsetzung erfolgt in einem unter den Reaktionsbedingungen inerten organischen Lösungsmittel, beispielsweise einem niederen Alkohol wie Methanol oder Äthanol, einem aromatischen Kohlenwasserstoff, einem offenen oder cyclischen Äther oder einem halogenierten Kohlenwasserstoff. Falls $R_2$ niederes Alkyl bedeutet, entstehen bei der Umsetzung Gemische von isomeren Verbindungen, worin $R_1$ in 1- oder 2-Stellung angeordnet ist. Das Verhältnis von 1-Alkyl zu 2-Alkyl-Verbindungen kann je nach Art der Ausgangsstoffe und des verwendeten Lösungsmittels variieren. Isomeren-Gemische aus 1- und 2-Alkyl-Verbindungen können auf an sich bekannte Weise durch fraktionierte Kristallisation oder durch Chromatographie getrennt werden.

Säuren und Ester der Formel II sind bekannt, beispielsweise aus der europäischen Patentanmeldung 7019, oder können nach an sich bekannten Verfahren, beispielsweise den in der europäischen Patentanmeldung 7019 beschriebenen Verfahren, hergestellt werden.

So können zur Herstellung von Säuren der Formel II Verbindungen der Formel VI auf an sich bekannte Weise mit Verbindungen der Formel XV

(siehe Formel XV)

worin $R_5$, $R_6$ und Hal obige Bedeutung besitzen und $R_{10}$ niederes Alkoxycarbonyl oder Cyano bedeutet, umgesetzt werden zu (5-Phenylpyrazol-3-oxy)-essigsäureestern und -nitrilen der Formel XVI

(siehe Formel XVI)

worin $R_1$, $R_2$, $R_3$, $R_4$, $R_5$, $R_6$ und $R_{10}$ obige Bedeutung besitzen, und diese anschliessend hydrolysiert werden. Die Umsetzung der Verbindungen der Formel VI mit den Verbindungen der Formel XV kann nach an sich zur Alkylierung am Sauerstoffatom in 3-Stellung von 5-Phenylpyrazolin-3-onen bekannten Verfahren unter basischen Bedingungen, beispielsweise unter den für Verfahrensvariante c angegebenen Bedingungen, durchgeführt werden. Zweckmässigerweise wird die Reaktion in Dimethylformamid in Gegenwart von Kaliumcarbonat ausgeführt. Im allgemeinen werden Ester der Formel XV eingesetzt. Falls $R_5$ und $R_6$ Wasserstoff bedeutet, erweisen sich Nitrile der Formel XV als vorteilhaft. Sofern der Phenylring einen freien Hydroxy-Substituenten besitzt, ist es zweckmässig, diesen in an sich bekannter Weise vor der Umsetzung mit einer Schutzgruppe zu versehen, welche anschliessend leicht wieder abspaltbar ist unter Bedingungen, unter denen die Äthergruppe in 3-Stellung des Pyrazolgerüstes und die Amidgruppe in der Seitenkette der Verbindungen unter Formel I nicht angegriffen werden.

Gewünschtenfalls kann in Verbindungen der Formel XVI, worin $R_2$ Wasserstoff bedeutet, eine Alkylgruppe $R_2$ auf an sich bekannte Weise durch Alkylierung unter basischen Bedingungen eingeführt werden.

Verbindungen der Formel XVI können auf an sich bekannte Weise zu den entsprechenden Säuren der Formel II hydrolysiert und diese in an sich bekannter Weise in weitere reaktionsfähige Säurederivate überführt werden. Die Hydrolyse erfolgt vorzugsweise unter alkalischen Bedingungen, beispielsweise durch Behandeln mit wässriger Alkalimetallhydroxydlösung, gegebenenfalls in Gegenwart eines mit Wasser mischbaren organischen Lösungsmittels. Die Überführung der freien Säuren in reaktive Säurederivate erfolgt ebenfalls auf an sich bekannte Weise. So können Säurehalogenide der Formel II z.B. durch Umsetzung der Säuren mit einem anorganischen Säurehalogenid, beispielsweise Thionylchlorid, erhalten werden. Gewünschtenfalls kann die Umsetzung in Gegenwart von Pyridin oder einer anderen tertiären organischen Base durchgeführt werden. Gemischte Säureanhydride können z.B. durch Umsetzung der Säuren mit einer äquivalenten Menge eines anderen Säurechlorides, beispielsweise eines Sulfonsäurechlorides wie Methansulfonsäurechlorid, in Gegenwart eines säurebindenden Mittels, vorzugsweise einer tertiären organische Base wie Triäthylamin, erhalten werden.

Verbindungen der Formel II, worin $R_2$ Wasserstoff bedeutet, können während der vorstehend beschriebenen Herstellungsverfahren teilweise zu den entsprechenden cyclischen Verbindungen der Formel III kondensiert werden, so dass die erhaltenen Verbindungen der Formel II je nach Reaktionsbedingungen wechselnde Mengen an Verbindungen der Formel III

als Nebenprodukt enthalten können. Da Verbindungen der Formeln II und III in gleicher Weise mit einem Amin weiter umgesetzt werden können, erübrigt sich die Auftrennung etwaiger Gemische aus Verbindungen der Formeln II und III.

Reine Verbindungen der Formel III können durch cyclisierende Kondensation von entsprechenden reaktionsfähigen Säurederivaten der Formel II, insbesondere von gemischten Anhydriden der Formel II, erhalten werden. Die Kondensation erfolgt beispielsweise, wenn die Lösung eines gemischten Anhydrids der Formel II in einem inerten organischen Lösungsmittel, z.B. einem halogenierten Kohlenwasserstoff wie Methylenchlorid, bei Raumtemperatur stehen gelassen wird.

Verbindungen der allgemeinen Formel IV können auf an sich bekannte Weise aus Verbindungen der allgemeinen Formel II erhalten werden, indem man diese mit Aminoalkoholen der Formel X

(siehe Formel X)

worin $R_7$ und Z obige Bedeutung besitzen, umsetzt und anschliessend in den erhaltenen Reaktionsprodukten die endständige Hydroxygruppe der 3-Seitenkette auf an sich bekannte Weise in eine aminolytisch abspaltbare Gruppe X überführt. Die Umsetzung der Verbindungen der Formel II mit den Verbindungen der Formel X kann unter zur Amidbildung üblichen Bedingungen beispielsweise den vorstehend für die Umsetzung von Verbindungen der Formel II mit Verbindungen der Formel VIII beschriebenen Bedingungen, erfolgen. Bei der Umsetzung findet überwiegend die gewünschte Amidbildung statt. Die endständige Hydroxygruppe in der Seitenkette des gebildeten Amids kann auf an sich bekannte Weise in eine aminolytisch abspaltbare Gruppe überführt werden, beispielsweise mit gebräuchlichen Halogenierungsmitteln, wie z.B. Thionylchlorid, Phosphoroxychlorid oder Phosphortribromid umgesetzt werden, um Verbindungen der Formel IV zu erhalten, worin X Halogen bedeutet; oder die endständige Hydroxygruppe kann nach an sich bekannten Methoden verestert werden, beispielsweise mit einem entsprechenden Säurehalogenid umgesetzt werden, um Verbindungen der Formel IV zu erhalten, worin X einen reaktiven Säurerest, insbesondere einen der vorgenannten Sulfonsäurereste, bedeutet. Bei diesen Umsetzungen reagiert die endständige Hydroxygruppe bevorzugt vor einer allfällig in der Gruppe Z enthaltenen sekundären Hydroxygruppe. Zur Herstellung von Verbindungen der Formel IV, worin X Halogen bedeutet, können die Verbindungen der Formel II auch direkt mit den entsprechenden Halogenalkylaminen umgesetzt werden.

Verbindungen der Formel VII können auf an sich bekannte Weise hergestellt werden durch Umsetzung von entsprechenden α-Halogencarbonsäuren, bzw. deren reaktiven Derivaten mit Diaminen der Formel VIII unter zur Amidbildung üblichen Bedingungen.

Verbindungen der Formel IX können erhalten werden indem man Verbindungen der Formel II, insbesondere Säurechloride der Formel II, mit Aminen der Formel XI

(siehe Formel XI)

worin $R_7$ obige Bedeutung besitzt, auf an sich bekannte Weise umsetzt.

Die Verbindungen der Formel I und ihre pharmakologisch akzeptablen Säureadditionssalze besitzen interessante pharmakologische Eigenschaften, insbesondere herzrhythmisierende Wirkungen. Die neuen Verbindungen zeichnen sich durch ausgeprägte antiarrhythmische Wirkungen mit einem günstigen Wirkungsprofil und gute physiologische Verträglichkeit aus. Insbesondere weisen die Verbindungen die Eigenschaft auf, die elektrische Reizschwelle zur Auslösung von Rhythmusstörungen am Herzen zu erhöhen.

Die zu verwendenden Dosen variieren naturgemäss je nach Art der verwendeten Substanz, der Applikationsart und des zu behandelnden Zustandes. Im allgemeinen werden jedoch befriedigende Ergebnisse in Tierversuchen mit Dosen von 0,01 bis 100 mg/kg Körpergewicht erhalten.

Die antiarrhythmischen Wirkungen der Verbindungen lassen sich in pharmakologischen Testmethoden am Tier nachweisen.

1. Bestimmung der Hemmwirkung gegenüber durch Chloroforminhalation in Mäusen hervorgerufenem Kammerflimmern.

Die Wirkung der Verbindungen auf die durch zu schnellem Atemstillstand führende Chloroforminhalation hervorgerufene ventrikuläre Fibrillation in Mäusen wird nach der Methode von Lawson (J. Pharmacol. Exp. Ther. 160, 22-23) bestimmt.

In dieser Versuchsanordnung kann gleichzeitig auch die minimale toxische Dosis bestimmt werden. Weiblichen Mäusen von 17 bis 24 g Körpergewicht werden die Testsubstanzen in 0,9%iger NaCl-Lösung i.p. verabreicht. Die Tiere werden einzeln in Glasbechern gehalten, wo sie auf mögliche toxische Symptome beobachtet werden. 10 min nach Verabreichung der Testsubstanzen werden die Tiere in bedeckte 300 ml Glasbecher umgesetzt, welche einen mit ungefähr 20 ml Chloroform getränkten Wattebausch enthalten. Sofort nach Eintreten des Atemstillstands werden die Tiere aus dem Glasbecher genommen und Herzrhythmus und -frequenz werden beobachtet. Es wird bestimmt, welcher Prozentsatz der Tiere durch die verabreichte Dosis der Testsubstanz gegen das Kammerflimmern geschützt wird.

2. Bestimmung der rechtsventrikulären elektrischen Flatterschwelle am Meerschweinchenherzen.

Die Prüfung der Fähigkeit der Substanzen, die elektrische Reizschwelle zu erhöhen, wird an männlichen Albino Pirbrigt-white Meerschweinchen mit 250 bis 450 g Körpergewicht durchgeführt. Die Tiere werden durch i.p. Applikation von 1,50 g/kg Urethan narkotisiert und in Rückenlage auf einer thermostatisierten Wärmebank plaziert. Die Prüfsubstanzen werden über eine in die Vene eingebundene Kanüle verabreicht. Jedes Tier erhält eine Folge von ansteigenden Dosen. Das Elektrokardiogramm wird mit einer bipolaren Ableitung (subkutane Nadelelektroden auf beiden Seiten des Thorax) abgenommen und auf einem Oszilloskop beobachtet. Über eine bipolare Saugelektrode, welche über die Vena jugularis in den rechten Ventrikel eingeführt ist, wird eine intrakardiale Stimulation durch Serien rechteckiger Stromstösse (Dauer 1 ms, Frequenz 50 Hz, Stimulator der Firma Hugo Sachs Elektronik) erzeugt. Die Stromstärke wird innerhalb von 5 bis 15 Sekunden bis zum Auftreten von Kammerflattern erhöht. Die Stromstärke (in µA), bei welcher Kammerflattern beobachtet wird, wird als rechtsventrikuläre Flatterschwelle definiert. Die Flatterschwelle der einzelnen Tiere wird vor Applikation der 1. Dosis sowie nach jeder weiteren verabreichten Dosis der Prüfsubstanz gemessen. Als $ED_{150}$% wird diejenige Dosis definiert, welche im Mittel eine Erhöhung der Flatterschwelle um 50% verursacht.

In der nachstehenden Tabelle werden mit den vorstehend beschriebenen Testmethoden erhaltene Ergebnisse wiedergegeben. Die für die Verbindungen der Formel I angegebenen Beispielsnummern beziehen sich auf die nachstehenden Herstellungsbeispiele.

| Beispiel Nr. | minimale toxische Dosis mg/kg Maus i.p. | Hemmwirkung gegenüber Chloroform-induziertem Kammerflimmern in Mäusen Dosis mg/kg | % geschützte Tiere | Erhöhung der elektr. Reizschwelle am Meerschweinchen $ED_{150}$% i.v. µmol/kg |
|---|---|---|---|---|
| 1 | 200 | 25 | 100 | 1,4 |
| 3 | 200 | 25 | 67 | 0,42 |
| 9 | 200 | 50 | 33 | 2,6 |
| 16 | 200 | 50 | 100 | 3,0 |
| 18 | 100 | 10 | 100 | 0,78 |
| 25 | | | | 1,2 |
| 27 | | | | 1,5 |
| 28 | | | | 2,0 |
| 30 | 200 | 25 | 67 | 1,9 |
| 31 | 100 | 25 | 67 | 1,1 |
| 38 | 200 | 50 | 100 | 3,0 |
| 40 | 100 | 25 | 100 | 0,75 |
| 41 | 200 | 52 | 100 | 1,5 |
| 55 | >200 | 100 | 100 | 2,2 |
| 56 | | | | 2,6 |
| 64 | >200 | 100 | 100 | 1,7 |
| 59 | 200 | 50 | 100 | 2,6 |
| 68 | | | | 2,7 |

Aufgrund der vorstehend beschriebenen Wirkungen eignen sich die Verbindungen der Formel I und ihre pharmakologisch akzeptablen Säureadditionssalze als Arzneimittel zur Behandlung und Prophylaxe von Herzrhythmusstörungen.

Als Heilmittel können die Verbindungen der Formel I und ihre physiologisch verträglichen Säureadditionssalze mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssiger Verdünnungsmittel wie z.B. Wasser, fetten Ölen oder flüssigen Paraffinen.

Die nachfolgenden Beispiele sollen die Herstellung der neuen Verbindungen der Formel I näher erläutern, jedoch den Umfang der Erfindung in keiner Weise beschränken.

Die Strukturen der neuen Verbindungen wurden durch spektroskopische Untersuchungen, insbesondere durch Analyse der NMR-, Massen-, IR- und/oder UV-Spektren gesichert.

*Beispiel 1*

*3-[2-(3-Diäthylaminopropylaminocarbonyl)-propyl-2-oxy]-5-phenylpyrazol*

A) 6,2 ml Benzoylessigsäureäthylester werden mit 3,7 ml Äthanol versetzt. Zu der Lösung werden unter Eiskühlung 3,3 ml 80%iges Hydrazinhydrat zugetropft und das Reaktionsgemisch 12 Stunden stehengelassen. Anschliessend wird das ausgefallene 5-Phenylpyrazolin-3-on abfiltriert und zunächst mit wenig Äthanol und dann mit Diäthyläther gewaschen.

Schmelzpunkt 236 bis 240°C, Ausbeute 5,5 g.

B) 5 g 5-Phenylpyrazolin-3-on werden in 50 ml Dimethylformamid gelöst. Zu der Lösung werden 1,5 g einer ca. 50%igen öligen Natriumhydrid Zubereitung portionsweise zugesetzt. 15 Minuten später werden 5,7 ml 2-Brom-2-methylpropionsäureäthylester zugetropft und das Reaktionsgemisch 24 Stunden bei einer Temperatur von 80°C gerührt. Anschliessend wird das Lösungsmittel unter vermindertem Druck abgedampft und der verbleibende Rückstand wird in Methylenchlorid suspendiert. Es wird von den ausgefallenen Natriumbromidkristallen abfiltriert, die Lösung mit Wasser gewaschen und die wässrigen Waschwasser werden nochmals mit Methylenchlorid extrahiert. Die vereinigten Methylenchloridphasen werden über Magnesiumsulfat getrocknet und eingeengt. Das erhaltene Rohprodukt wird durch Säulenchromatographie über Kieselgel unter Verwendung von Hexan/Äthergemischen als Elutionsmittel gereinigt. Es werden 4,9 g kristallines 3-(2-Äthoxycarbonylpropyl-2-oxy)-5-phenylpyrazol erhalten.

Schmelzpunkt 79°C.

C) 42 g 3-(2-Äthoxycarbonylpropyl-2-oxy)-5-phenylpyrazol werden in 200 ml Äthanol gelöst, die Lösung mit 103 ml 20%iger Natronlauge versetzt und das Reaktionsgemisch bis zur vollständigen Umsetzung unter Rückfluss erhitzt. Das Äthanol wird weitgehend abdestilliert und das verbleibende wässrige Reaktionsgemisch wird unter Eiskühlung mit 20%iger Salzsäure bis auf pH 1 angesäuert. Das ausgefallene 3-(2-Hydroxycarbonylpropyl-2-oxy)-5-phenylpyrazol wird abfiltriert.

Schmelzpunkt 152 bis 155°C, Ausbeute 36,5 g.

D) 493 g 3-(2-Hydroxycarbonylpropyl-2-oxy)-5-phenylpyrazol werden in 4 ml trockenem Methylenchlorid suspendiert und mit 0,55 ml Triäthylamin versetzt. Nach 10 Minuten wird das Reaktionsgemisch auf −30°C Badtemperatur gekühlt und zur Bildung eines gemischten Anhydrids tropfenweise mit 0,16 ml Methansulfonsäurechlorid versetzt und

noch eine Stunde bei $-30°C$ gerührt. Das das gebildete 2-Methyl-2-[(5-phenylpyrazol-3-yl)-oxy]-propionsäuremethansulfonsäureanhydrid enthaltende Reaktionsgemisch wird sofort weiterverarbeitet.

E) Zu dem unter D) hergestellten das gemischte Anhydrid enthaltenden Reaktionsgemisch wird eine Spatelspitze 4-Pyrrolidinopyridin zugeben und anschliessend werden 0,32 ml 3-Diäthylaminopropylamin zugetropft und das Reaktionsgemisch langsam auf Raumtemperatur erwärmt und noch 2,5 Stunden gerührt. Dann wird mit 5 ml Methylenchlorid versetzt und 2 mal mit verdünnter mit verdünnter Natriumcarbonatlösung gewaschen. Die organische Phase wird abgetrennt, getrocknet und eingeengt. Es verbleiben 649 mg der Titelverbindung als ölige Base [IR-Spektrum (als Film): 3220 $cm^{-1}$, 1650 $cm^{-1}$, 1530 $^{-1}$]. Die Base wird in Aceton gelöst und durch Zugabe einer Lösung von 232 mg Maleinsäure in Aceton in ihr Hydrogenmaleinat überführt. Es werden 644,5 mg 3-[2-(Diäthylaminopropylaminocarbonyl)-propyl-2-oxy]-5-phenylpyrazol-hydrogenmaleinat erhalten.

Schmelzpunkt 135°C.

*Beispiel 2*

*3-[2-(3-Diäthylaminopropylaminocarbonyl)-propyl-2-oxy]-5-phenylpyrazol*

Zu einem analog Beispiel 1D hergestellten das gemischte Anhydrid aus 493 mg 3-[2-Hydroxycarbonylpropyl-2-oxy]-5-phenylpyrazol und Methansulfonsäure enthaltenden Reaktionsgemisch werden 0,32 ml 3-Diäthylaminopropylamin zugetropft und das Reaktionsgemisch langsam auf Raumtemperatur erwärmt und noch 2,5 Stunden gerührt. Dann wird wie in Beispiel 1E beschrieben aufgearbeitet. Es werden 515 mg der Titelverbindung als ölige Base erhalten. Diese wird wie in Beispiel 1E beschrieben in ihr Hydrogenmaleinat überführt. Schmelzpunkt 135°C.

*Beispiel 3*

*3-[2-(3-Diäthylamino-2-hydroxypropylaminocarbonyl)-propyl-2-oxy]-5-phenylpyrazol*

A) 4,7 g 5-Phenylpyrazolin-3-on und 4,1 g wasserfreies Kaliumcarbonat werden in 60 ml Dimethylformamid suspendiert. Die Suspension wird ca. 10 Min. auf 100°C erwärmt, dann auf 60°C abgekühlt und tropfenweise mit 4,8 ml 2-Brom-2-methylpropionsäureäthylester versetzt. Es wird 4 Stunden bei 100°C gerührt und anschliessend das Lösungsmittel unter vermindertem Druck abgedampft und der Rückstand in 150 ml Wasser gelöst. Die wässrige Lösung wird zweimal mit je 150 ml Diäthyläther extrahiert und die organische Phase über Magnesiumsulfat getrocknet und eingeengt. Es werden 6,9 g rohes 3-(2-Äthoxycarbonylpropyl-2-oxy)-5-phenylpyrazol als Öl erhalten. Dieses wird aus Essigsäureäthylester kristallisiert, Schmelzpunkt 78,5°C. Anschliessend wird das Produkt wie in Beispiel 1C beschrieben zu 3-(2-Hydroxycarbonylpropyl-2-oxy)-5-phenylpyrazol hydrolysiert.

B) 9,85 g 3-(2-Hydroxycarbonylpropyl-2-oxy)-5-phenylpyrazol werden nach dem in Beispiel 1D beschriebenen Verfahren mit Methansulfonsäurechlorid zu dem gemischten Anhydrid umgesetzt und dieses in situ mit 3-Diäthylamino-2-hydroxypropylamin analog Beispiel 1E umgesetzt. Das Reaktionsgemisch wird wie in Beispiel 1E beschrieben aufgearbeitet und das erhaltene Rohprodukt wird zur weiteren Reinigung an einer Kieselgelsäule chromatographiert unter Verwendung eines Äther/Methanol-Gemisches als Elutionsmittel. Man erhält 10,5 g der Titelverbindung als ölige Base. Diese wird in Aceton gelöst und durch Zugabe einer Lösung von 3,25 g Maleinsäure in Aceton in ihr Hydrogenmaleinat überführt. Es werden 10,6 g 3-[3-Diäthylamino-2-hydroxypropylaminocarbonyl]-propyl-2-oxy[-5-phenylpyrazolhydrogenmaleinat erhalten, Schmelzpunkt 160°C.

*Beispiel 4*

*3-(2-Aminoäthylaminocarbonylpropyl-2-oxy)-5-phenylpyrazol*

8,6 g 3-(2-Hydroxycarbonylpropyl-2-oxy)-5-phenylpyrazol werden in 60 ml Methylenchlorid gelöst und mit 9,7 ml Triäthylamin versetzt. Nach 10 Minuten kühlt man auf $-30°C$ Badtemperatur, versetzt das Gemisch tropfenweise mit 2,74 ml Methansulfonsäurechlorid und rührt noch eine Stunde bei $-30°C$. Die das gebildete gemischte Anhydrid enthaltende Lösung wird in einen auf $-30°C$ temperierten Tropfrichter überführt und innerhalb 1,5 Stunden zu 45 ml Äthylendiamin getropft und zur Vervollständigung der Reaktion anschliessend noch eine Stunde bei Raumtemperatur gerührt. Anschliessend wird das Reaktionsgemisch mit Methylenchlorid verdünnt und mit 20%iger Sodalösung gewaschen. Die organische Phase wird getrocknet und eingedampft. Das verbleibende Rohprodukt wird durch Chromatographie an Aluminiumoxid unter Verwendung von Methylenchlorid/Methanol als Elutionsmittel gereinigt. Zur Überführung in ihr Hydrogenmaleinat wird die chromatographisch gereinigte Titelverbindung in Aceton und mit einer Lösung von 1,57 g Maleinsäure in Aceton versetzt und die Lösung eingedampft. Der Rückstand wird mit Wasser aufgenommen, die wässrige Phase zweimal mit Äther extrahiert und anschliessend durch Zusatz von Natriumcarbonatlösung alkalisiert und die aus dem Maleinat wieder freigesetzte Titelbase mit Äther extrahiert. Beim Eindampfen des Ätherextraktes werden 1,7 g der Titelverbindung erhalten. Diese werden zusammen mit 1,1 g Maleinsäure in Isopropanol gelöst. Die Lösung wird in Äther eingetropft. Hierbei fällt das 3-(2-Aminoäthylaminocarbonylpropyl-2-oxy)-5-phenylpyrazol-hydrogenmaleinat aus. Es wird abfiltriert und mit Äther gewaschen.

Schmelzpunkt 128 bis 135°C, Ausbeute 1,3 g.

*Beispiel 5*

*3-(Diäthylaminoäthylaminocarbonylmethoxy)-5-phenylpyrazol*

A) 40 g 5-Phenylpyrazolin-3-on werden analog dem in Beispiel 1B beschriebenen Verfahren in 120 ml Dimethylformamid zunächst durch Umsetzung mit 5,6 g einer 80%igen öligen Natriumhydridzubereitung deprotonisiert und anschliessend mit 11 ml Chloracetonitril zu 3-Cyanomethoxy-5-phenylpyrazol umgesetzt. Fp.: 143°C.

B) 25,2 g 3-Cyanomethoxy-5-phenylpyrazol werden in 200 ml Äthanol gelöst und mit 50 ml 20%iger Natronlauge versetzt. Das Reaktionsgemisch wird eine Stunde am Rückfluss erhitzt. Nach dem Abkühlen wird mit 20%iger wässriger Salzsäure auf pH 1 angesäuert. Das ausgefallene 3-Hydroxycarbonylmethoxy-5-phenylpyrazol wird abfiltriert. Fp.: 193°C, Ausbeute 22,3 g.

C) 4,36 g 3-Hydroxycarbonylmethoxy-5-phenylpyrazol werden nach dem in Beispiel 1D und E beschriebenen Verfahren durch Umsetzung mit Methansulfonsäurechlorid in das gemischte Säureanhydrid überführt und dieses in situ mit 2-Diäthylaminoäthylamin umgesetzt. Das Reaktionsgemisch wird wie in Beispiel 1E beschrieben aufgearbeitet. Das erhaltene 3-(Diäthylaminoäthylaminocarbonylmethoxy)-5-phenylpyrazol wird aus Essigsäureäthylester kristallisiert. Schmelzpunkt 100 bis 101°C, Ausbeute 4,6 g.

*Beispiel 6*

*3-(2-Morpholinoäthylaminocarbonylmethoxy)-5-phenylpyrazol*

Zu 3,06 g 1-Methyl-2-chlorpyridiniumjodid im Methylenchlorid werden nacheinander 1,33 ml N-(2-Aminoäthyl)-morpholin, 2,2 g 3-Hydroxycarbonylmethoxy-5-phenylpyrazol und 4,3 ml Triäthylamin gegeben. Anschliessend wird das Reaktionsgemisch 3 Stunden unter Rückfluss zum Sieden erhitzt. Nach dem Abkühlen wird mit wässriger Sodalösung gewaschen, die organische Phase eingedampft und das erhaltene Rohprodukt chromatographisch gereinigt. Es werden 2,3 g 3-(2-Morpholinoäthylaminocarbonylmethoxy)-5-phenylpyrazol als ölige Base erhalten.

IR-Spektrum (als Film): 3205 $cm^{-1}$, 1660 $cm^{-1}$.

*Beispiel 7*

*3-[2-(3-Diäthylaminopropylaminocarbonyl)-propyl-2-oxy]-5-(3,4-dichlorphenyl)-pyrazol*

13,7 g 3-(2-Äthoxycarbonylpropyl-2-oxy)-5-(3,4-dichlorphenyl)-pyrazol werden zu 30 ml 3-Diäthylaminopropylamin gegeben und das Reaktionsgemisch wird unter Stickstoff 18 Stunden auf 180°C erhitzt. Nach Beendigung der Reaktion wird überschüssiges Amin im Vakuum abdestilliert. Dann wird die erhaltene rohe Titelverbindung im Exsikkator über konzentrierter Schwefelsäure getrocknet. Es verbleiben 16,2 g 3-[2-(3-Diäthylaminopropylaminocarbonyl)-propyl-2-oxy]-5-(3,4-dichlorphenyl)-pyrazol als ölige Base. Zur weiteren Reinigung wird die Verbindung in Äther gelöst und die ätherische Lösung über Kieselgel filtriert.

IR-Spektrum (als Film): 3200 $cm^{-1}$, 1645 $cm^{-1}$, 1530 $cm^{-1}$.

*Beispiel 8*

*3-[2-(3-Morpholinopropylaminocarbonyl)-propyl-2-oxy]-5-phenylpyrazol*

9 g 3-(2-Hydroxycarbonylpropyl-2-oxy)-5-phenylpyrazol werden in Methylenchlorid gelöst und die Lösung unter Eiskühlung tropfenweise mit 5,6 ml Triäthylamin versetzt. Nach 30 Minuten Rühren werden 3,6 ml Chlorameisensäureäthylester tropfenweise zugesetzt und das Reaktionsgemisch wird noch eine Stunde bei Raumtemperatur gerührt. Das das gebildete 3-(2-Äthoxycarbonyloxycarbonylpropyl-2-oxy)-5-phenylpyrazol enthaltende Reaktionsgemisch wird anschliessend unter Eiskühlung zu einer Lösung von 8 ml N-(3-Aminopropyl)-morpholin in Methylenchlorid getropft. Das Gemisch wird noch 1 Stunde unter Eiskühlung und 1 weitere Stunde bei Raumtemperatur gerührt, dann wird mit verdünnter Sodalösung gewaschen, die organische Phase getrocknet und eingedampft. Der Rückstand wird in 75 ml Äthanol gelöst. Es werden 35 ml 20%ige Natronlauge zugegeben und 3 Stunden unter Rückfluss erhitzt. Anschliessend wird auf das halbe Volumen eingeengt, mit Wasser verdünnt und mit Methylenchlorid extrahiert. Das aus der Methylenchloridphase erhaltene Rohprodukt wird durch Chromatographie an Kieselgel unter Verwendung eines Äther/Methanolgemisches gereinigt. Es werden 5,9 g 3-[2-(3-Morpholinopropylaminocarbonyl)-propyl-2-oxy]-5-phenylpyrazol als ölige Base erhalten.

IR-Spektrum (als Film): ~3240 $^{-1}$, 1650 $cm^{-1}$, 1530 $cm^{-1}$.

*Beispiel 9*

*3-[2-(3-Diäthylaminopropylaminocarbonyl)-propyl-2-oxy]-1-methyl-5-phenylpyrazol*

A) 20,5 Propiolsäuremethylester werden in 150 ml Toluol gelöst. Zu der Lösung werden unter Eiskühlung 6,7 ml Methylhydrazin zugetropft. Nach 12-stündigem Stehen wird die Lösung eingedampft. Als Rückstand wird ein Gemisch aus 1-Methyl-5-phenylpyrazolin-3-on und 2-Methyl-5-phenylpyrazolin-3-on erhalten. Dieses rohe isomere Gemisch wird an Kieselgel chromatographiert. Als Elutionsmittel wird Äther verwendet, welchem steigende Mengen Methanol zugesetzt werden. Aus dem Eluat wird das unpolarere 1-Methyl-5-phenylpyrazolin-3-on isoliert. Fp.: 160 - 161°C, Ausbeute 6,3 g.

B) 4,5 g 1-Methyl-5-phenylpyrazolin-3-on werden wie in Beispiel 3A beschrieben zu 3-(2-Äthoxycarbonylpropyl-2-oxy)-1-methyl-5-phenylpyrazol umgesetzt und dieses ohne Reinigung weiter verseift zu 3-(2-Hydroxycarbonylpropyl-2-oxy)-1-methyl-5-phenylpyrazol.

C) Das vorstehend erhaltene rohe 3-(2-Hydroxylcarbonylpropyl-2-oxy)-1-methyl-5-phenylpyrazol wird wie in Beispiel 1D beschrieben durch Umsetzen mit Methansulfonsäurechlorid in das gemischte Anhydrid überführt und dieses mit 3-Diäthylaminopropylamin umgesetzt. Das Reaktionsgemisch wird wie in Beispiel 1E beschrieben aufgearbeitet und die Titelverbindung als ölige Base isoliert.

IR-Spektrum (als Film): ~3340$^{-1}$, 1669 $cm^{-1}$, 1545 $cm^{-1}$.

Die Titelverbindung wird anschliessend wie in Beispiel 1E beschrieben in ihr Hydrogenmaleinat überführt. Es werden 3,27 g 3-[2-(3-Diäthylaminopropylaminocarbonyl)-propyl-2-oxy]-1-methyl-5-phenylpyrazol-hydrogenmaleinat als Öl erhalten.

*Beispiel 10*

*3-[2-(3-Diäthylaminopropylaminocarbonyl)-propyl-2-oxy]-5-phenylpyrazol*

A) 2,3 g 3-(2-Hydroxycarbonylpropyl-2-oxy)-5-phenylpyrazol (Herstellung s. Beispiel 1C) werden in 50 ml trockenem Methylenchlorid suspendiert und mit 2,6 ml Triäthylamin versetzt. Nach 10 Minuten wird das Reaktionsgemisch auf -30°C Badtemperatur gekühlt, zur Bildung eines gemischten Anhydrides nach der in Beispiel 1D beschriebenen Methode tropfenweise mit 0,72 ml Methansulfonsäurechlorid versetzt und noch eine Stunde bei -30°C gerührt. Anschliessend wird die das gemischte Anhydrid enthaltende Reaktionslösung zur cyclisierenden Kondensation des gemischten Anhydrides zu dem entsprechenden 2,2-Dimethyl-6-phenyl-pyrazolo[5,1-b]oxazolin-3-on langsam auf Raumtemperatur erwärmt und bis zur vollständigen Umsetzung bei Raumtemperatur gerührt. Sodann wird das Reaktionsgemisch mit Natriumhydrogencarbonatlösung gewaschen, die organische Phase abgetrennt, getrocknet und eingeengt. Das erhaltene 2,2-Dimethyl-6-phenyl-pyrazolo[5,1-b]oxazolin-3-on wird aus einem Gemisch von Essigsäureäthylester und Diäthyläther umkristallisiert, Schmelzpunkt 110°C.

B) 230 mg 2,2-Dimethyl-6-phenyl-pyrazolo[5,1-b]oxazolin-3-on und 0,16 ml 3-Diäthylaminopropylamin werden in 1 ml trockenem Tetrahydrofuran gelöst und die Lösung unter Stickstoffatmosphäre bei Raumtemperatur ca. 50 Stunden gerührt. Anschliessend wird im Vakuum eingedampft und der Rückstand in Diäthyläther gelöst. Die Lösung wird mit konzentrierter Natriumcarbonatlösung gewaschen, die organische Phase abgetrennt, getrocknet und eingedampft. Es werden 360 mg 3-[2-(3-Diäthylaminopropylaminocarbonyl)-propyl-2-oxy]-5-phenylpyrazol als ölige Base erhalten. Diese wird wie in Beispiel 1E beschrieben in ihr Hydrogenmaleinat überführt, Schmelzpunkt 135°C.

*Beispiel 11*

*3-[2-(3-Diäthylaminopropylaminocarbonyl)-propyl-2-oxy]-5-phenylpyrazol*

230 mg 2,2-Dimethyl-6-phenyl-pyrazolo[5,1-b] oxazolin-3-on (Herstellung s. Beispiel 10A) werden in ca. 2 ml 3-Diäthylaminopropylamin unter Stickstoff gelöst und die Lösung 2 Stunden bei Raumtemperatur stehengelassen. Anschliessend wird das überschüssige Amin im Vakuum abdestilliert. Die verbleibende rohe Titelverbindung wird zur Entfernung basischer Verunreinigungen im Exsikkator über konzentrierter Schwefelsäure getrocknet. Es werden 360 mg 3-[2-(3-Diäthylaminopropylaminocarbonyl)-propyl-2-oxy]-5-phenylpyrazol als ölige Base erhalten. Diese wird wie in Beispiel 1E beschrieben in ihr Hydrogenmaleinat überführt, Schmelzpunkt 135°C.

*Beispiel 12*

*3-[2-(2-Morpholinoäthylaminocarbonyl)-propyl-2-oxy]-2-methyl-5-phenylpyrazol*

A) 5 g 3-(2-Hydroxycarbonylpropyl-2-oxy)-2-methyl-5-phenylpyrazol werden in 40 ml trockenem Methylenchlorid suspendiert und mit 8,3 ml Triäthylamin versetzt. Nach 10 min wird das Reaktionsgemisch zur Bildung eines gemischten Anhydrids tropfenweise mit 1,48 ml Methansulfonsäurechlorid versetzt und noch 30 Minuten gerührt. Zu dem das gemischte Anhydrid enthaltenden Reaktionsgemisch wird dann eine Spatelspitze 4-Pyrrolidinopyridin zugegeben und anschliessend werden 3,9 g 2-Bromäthylaminhydrobromid portionsweise innerhalb von 30 min zugefügt. Das Reaktionsgemisch wird 3 Stunden stehengelassen, dann wird zunächst mit wässriger Zitronensäurelösung und danach mit Natriumcarbonatlösung gewaschen. Die organische Phase wird getrocknet und eingeengt. Als Rückstand erhält man 3,8 g 3-[2-(2-Bromäthylaminocarbonyl)-propyl-2-oxy]-2-methyl-5-phenylpyrazol, Schmelzpunkt 130°C.

B) 3,8 g (3-[2-(2-Bromäthylaminocarbonyl)-propyl-2-oxy]-2-methyl-5-phenylpyrazol werden in wenig Äthanol gelöst, und die Lösung wird innerhalb von 2 Stunden in 100 ml siedendes Morpholin getropft. Das Reaktionsgemisch wird weitere 30 min am Rückfluss erhitzt und anschliessend im Vakuum eingeengt. Der verbleibende Rückstand wird in Methylenchlorid aufgenommen und mit wässriger Zitronensäurelösung extrahiert. Die wässrige Phase wird abgetrennt, durch Zugabe von Natriumcarbonatlösung alkalisiert und mit Äther extrahiert. Die Ätherphase wird abgetrennt und eingedampft. Der verbleibende Rückstand wird im Exsikkator über Schwefelsäure getrocknet. Die so erhaltene rohe Titelverbindung wird durch Chromatographie an Kieselgel gereinigt. Es werden 2,8 g 3-[2-(2-Morpholinoäthylaminocarbonyl)-propyl-2-oxy]-2-methyl-5-phenylpyrazol erhalten, Schmelzpunkt 105°C.

IR-Spektrum (in KBr): ~3340 $cm^{-1}$, 1670 $cm^{-1}$, 1545 $cm^{-1}$.

*Beispiel 13*

*3-[2-(3-Diäthylaminopropylaminocarbonyl)-propyl-2-oxy]-5-phenylpyrazol*

A) 3,4 g 2-Brom-2-methylpropionsäure werden in 30 ml trockenes Methylenchlorid gegeben und mit 5,4 ml Triäthylamin versetzt. Nach 10 min wird das Reaktionsgemisch auf -30°C Badtemperatur gekühlt, zur Bildung eines gemischten Anhydrids tropfenweise mit 1,4 ml Methansulfonsäurechlorid versetzt und noch eine Stunde bei -30°C gerührt. Anschliessend werden eine Spatelspitze 4-Pyrrolidinopyridin und sodann 3,1 ml 3-Diäthylaminopropylamin zugetropft und das Reaktionsgemisch langsam auf Raumtemperatur erwärmt und noch 2,5 Stunden bei Raumtemperatur gerührt. Dann werden 40 ml Methylenchlorid zugegeben und das Gemisch zweimal mit verdünnter Natriumcarbonatlösung gewaschen. Die organische Phase wird abgetrennt, getrocknet und eingeengt. Es werden 3 g N-(3-Diäthylaminopropyl)-2-brom-2-methylpropionsäureamid als ölige Base erhalten.

IR-Spektrum (als Film): ~3310 $cm^{-1}$, 1660 $cm^{-1}$, 1530 $cm^{-1}$.

B) 1,6 g 5-Phenylpyrazolin-3-on und 2 g wasserfreies Kaliumcarbonat werden in 20 ml Dimethylformamid suspendiert. Die Suspension wird 10 min

lang auf 100°C erwärmt. Dann wird auf 60°C abgekühlt und zu dem Gemisch tropfenweise eine Lösung von 3 g N-(3-Diäthylaminopropyl)-2-brom-2-methylpropionsäureamid in 10 ml Dimethylformamid zugegeben. Das Reaktionsgemisch wird 4 Stunden bei 100°C gerührt. Anschliessend wird das Lösungsmittel unter vermindertem Druck abgedampft und der Rückstand in 50 ml Wasser gelöst. Die wässrige Lösung wird zweimal mit je 50 ml Diäthyläther extrahiert. Die vereinigten Ätherextrakte werden über Magnesiumsulfat getrocknet und eingeengt. Es werden 0,9 g 3-[2-(3-Diäthylaminopropylaminocarbonyl)-propyl-2-oxy]-5-phenylpyrazol als ölige Base erhalten.

IR-Spektrum (als Film): 3220 $cm^{-1}$, 1650 $cm^{-1}$, 1530 $cm^{-1}$.

Die Base wird wie in Beispiel 1E beschrieben in ihr Hydrogenmaleinat überführt, Schmelzpunkt 135°C.

*Beispiel 14*

*3-[2-(-Diäthylaminopropylaminocarbonyl)-propyl-2-oxy]-5-(4-hydroxyphenyl)-pyrazol*

160 mg 3-[2-(3-Diäthylaminopropylaminocarbonyl)-propyl-2-oxy]-5-(4-methoxyphenyl)-pyrazol werden mit 0,32 ml Essigsäureanhydrid und 0,7 ml 57%iger wässriger Jodwasserstoffsäure versetzt. Das Gemisch wird eine Stunde unter Rückfluss erhitzt und anschliessend vorsichtig in eiskalte Natriumcarbonatlösung gegeben und erschöpfend mit Diäthyläther extrahiert. Der Ätherextrakt wird über Magnesiumsulfat getrocknet und eingedampft. Die zurückbleibende rohe Titelverbindung wird durch Chromatographie an Kieselgel (Laufmittel: Essigsäureäthylester enthaltend 15% Triäthylamin) gereinigt. Es werden 15 mg 3-[2-(3-Diäthylaminopropylaminocarbonyl)-propyl-2-oxy]-5-(4-hydroxyphenyl)-pyrazol erhalten.

IR-Spektrum (als Film): 3220 $cm^{-1}$, 1655 $cm^{-1}$, ~1530 $cm^{-1}$.

Nach den in den vorstehenden Beispielen beschriebenen Verfahren können auch die in der folgenden Tabelle aufgeführten Verbindungen der Formel I hergestellt werden. Die in der Tabelle angeführten IR-Banden sind die charakteristischsten Banden der IR-Spektren in $cm^{-1}$ der jeweiligen freien Basen (sofern nicht anders angegeben als Film).

| Beispiel Nr. | $R_1$ | $R_2$ | $R_3$ | $R_4$ | $R_5$ | $R_6$ | $R_7$ | Z | $R_8$ | $R_9$ | Bemerkungen |
|---|---|---|---|---|---|---|---|---|---|---|---|
| 15 | H | 2-$CH_3$ | H | H | $CH_3$ | $CH_3$ | H | $CH_2$-CHOH-$CH_2$ | -$(CH_2)_5$- | | H-ma, öl; B, öl<br>IR: ~3360, 1665, 1545 |
| 16 | H | 2-$CH_3$ | H | H | $CH_3$ | $CH_3$ | H | n-$C_3H_6$ | $C_2H_5$ | $C_2H_5$ | H-ma, öl; B, öl<br>IR: 3330, 1660, 1540 |
| 17 | H | H | H | H | H | H | H | $C_2H_4$ | $CH_3$ | $CH_3$ | B, Fp. 123°C |
| 18 | $CH_3$ | H | H | H | $CH_3$ | $CH_3$ | H | $C_2H_4$ | $C_2H_5$ | $C_2H_5$ | H-ma, öl; B, öl<br>IR: ~3230, 1655, 1518 |
| 19 | H | H | H | H | $CH_3$ | $CH_3$ | H | $C_2H_4$ | $C_2H_4$-O-$C_2H_4$ | | B, Fp. 120°C |
| 20 | H | H | H | H | H | H | $CH_3$ | $C_2H_4$ | $CH_3$ | $CH_3$ | H-ma, Fp. 130°C |
| 21 | H | H | H | H | $CH_3$ | $CH_3$ | $CH_3$ | $C_2H_4$ | $CH_3$ | Ch3 | B, öl<br>IR: ~3220, ~1625 |
| 22 | H | H | H | H | $CH_3$ | $CH_3$ | $C_2H_5$ | $C_2H_4$ | $C_2H_5$ | $C_2H_5$ | H-fu, öl; B, öl<br>IR: ~3230, ~1630 |
| 23 | H | H | H | H | $CH_3$ | $CH_3$ | H | $C_2H_4$ | H | $CH(CH_3)_2$ | B, Fp. 133°C |
| 24 | H | H | H | H | H | H | H | n-$C_3H_6$ | $C_2H_5$ | $C_2H_5$ | B, Fp. 74-75°C |
| 25 | $CH_3$ | H | H | H | $CH_3$ | $CH_3$ | H | n-$C_3H_6$ | $C_2H_5$ | $C_2H_5$ | H-ma, FP. 90-95°C |
| 26 | H | H | H | H | $CH_3$ | $CH_3$ | H | n-$C_3H_6$ | n-$C_4H_9$ | n-$C_4H_9$ | B, öl<br>IR: ~3220, ~1655, ~1525 |
| 27 | H | H | H | H | $CH_3$ | $CH_3$ | H | n-$C_3H_6$ | -C-$(CH_2)_4$-<br>\|<br>$CH_3$ | | H-ma, Sch; B, öl<br>IR: ~3210, ~1650, 1525 |
| 28 | H | H | H | H | $CH_3$ | $CH_3$ | H | n-$C_3H_6$ | -$(CH_2)_5$- | | H-ma, öl; B, öl<br>IR: ~3200, ~1655, 1525 |
| 29 | $CH_3$ | H | H | H | $CH_3$ | $CH_3$ | H | n-$C_3H_6$ | $C_2H_4$-O-$C_2H_4$ | | B, Fp. 124°C |
| 30 | H | H | H | H | $CH_3$ | $CH_3$ | H | $CH_2$-$CH_2$-(1-$CH_3$-pyr-2) | | | H-ma, Sch; B, öl<br>IR: ~3210, ~1660, 1530 |

B = Base, öl = ölig, Sch. = Schaumharz, H-ma = Hydrogenmaleinat, H-fu = Hydrogenfumarat, H2-Cit- = Dihydrogencytrat

| Beispiel Nr. | $R_1$ | $R_2$ | $R_3$ | $R_4$ | $R_5$ | $R_6$ | $R_7$ | Z | $R_8$ | $R_9$ | Bemerkungen |
|---|---|---|---|---|---|---|---|---|---|---|---|
| 31 | $CH_3$ | H | H | H | $CH_3$ | $CH_3$ | H | $CH_2$-CHOH-$CH_2$ | $C_2H_5$ | $C_2H_5$ | H-ma, öl; B, Fp. 108°C |
| 32 | $CH_3$ | H | H | H | $CH_3$ | $CH_3$ | H | $CH_2$-CHOH-$CH_2$ | $C_2H_4$-O-$C_2H_4$ | | H-ma, Sch; B, FP. 148°C |
| 33 | H | H | H | H | $CH_3$ | $CH_3$ | H | $C_2$CHOH-$CH_2$ | $C_2H_4$-O-$C_2H_4$ | | H-ma, Sch; B, öl IR: ~3250, 1650, 1530 |
| 34 | H | H | 4-$CH_3$ | H | H | $C_2H_5$ | H | $C_2H_4$ | $C_2H_5$ | $C_2H_5$ | H-ma, öl; B, öl IR: ~3240, 1658, 1530 |
| 35 | H | H | 4-$CH_3$ | H | $C_2H_5$ | H | H | $C_2H_4$ | -$(CH_2)_5$- | | H-ma, Sch; B, öl IR: ~3240, 1658, ~1530 |
| 36 | H | H | 4-$CH_3$ | H | $C_2H_5$ | H | H | $C_2H_4$ | $C_2H_4$-O-$C_2H_4$ | | H-ma, öl; B, öl IR: ~3230, 1652, ~1530 |
| 37 | H | H | 4-$CH_3$ | H | $CH_3$ | $CH_3$ | H | n-$C_3H_6$ | $C_2H_5$ | $C_2H_5$ | H-ma, öl; B, öl IR: ~3220, 1652, ~1530 |
| 38 | H | H | 4-$CH_3$ | H | H | $C_2H_5$ | H | n-$C_3H_6$ | $C_2H_5$ | $C_2H_5$ | H-ma, öl; B, öl IR: ~3220, 1650, 1530 |
| 39 | H | H | 3-$CF_3$ | H | $CH_3$ | $CH_3$ | H | n-$C_3H_6$ | $C_2H_5$ | $C_2H_5$ | H-ma, Fp. 132°C |
| 40 | H | H | 2-F | H | -$(CH_2)_3$- | | H | n-$C_3H_6$ | $C_2H_5$ | $C_2H_5$ | H-ma, Fp. 107°C |
| 41 | H | H | 2-Cl | H | $CH_3$ | $CH_3$ | H | n-$C_3H_6$ | $C_2H_5$ | $C_2H_5$ | H-ma, öl; B, öl IR: ~3200, 1650, 1525 |
| 42 | H | H | 4-Cl | H | H | $C_2H_5$ | H | $C_2H_4$ | $C_2H_5$ | $C_2H_5$ | H-ma, öl; B, öl IR: ~3220, ~1653, ~1525 |
| 43 | H | H | 4-Cl | H | H | $C_2H_5$ | H | $C_2H_4$ | $C_2H_4$-O-$C_2H_4$ | | H-ma, Sch; B, Fp. 125-127°C |
| 44 | H | H | 4-Cl | H | H | $C_2H_5$ | H | n-$C_3H_6$ | $C_2H_5$ | $C_2H_5$ | H-ma, öl, B, Fp. 119°C |
| 45 | $CH_3$ | H | 4-Cl | H | $CH_3$ | $CH_3$ | H | n-$C_3H_6$ | $C_2H_4$-O-$C_2H_4$ | | H-ma, Sch; B, Fp. 141°C |
| 46 | $CH_3$ | H | 4-Cl | H | $CH_3$ | $CH_3$ | H | n-$C_3H_6$ | $C_2H_5$ | $C_2H_5$ | H-ma, Sch; B, öl IR: ~3220, ~1650, ~1525 |
| 47 | H | H | 4-Cl | H | H | $C_2H_5$ | H | $C_2H_4$ | -$(CH_2)_5$- | | B, Sch. IR*: 3210, 1655, 1530 |
| 48 | H | H | 4-Cl | H | H | $C_2H_5$ | H | $C_2H_4$ | -$(CH_2)_4$- | | B, Fp. 115°C |
| 49 | H | H | 4-Br | H | $CH_3$ | $CH_3$ | H | $C_2H_4$ | $C_2H_5$ | $C_2H_5$ | $H_2$-ma, öl; B, öl IR: 3230, 1655, ~1530 |
| 50 | H | H | 4-Br | H | $CH_3$ | $CH_3$ | H | n-$C_3H_6$ | $C_2H_5$ | $C_2H_5$ | H-ma, öl; B, öl IR: ~3200, ~1650, ~1530 |
| 51 | H | H | 4-$CH_3$O | H | $CH_3$ | $CH_3$ | H | $CH_2$-CHOH-$CH_2$ | $C_2H_5$ | $C_2H_5$ | H-cit, Sch; B, öl IR: ~3250, 1655, 1530 |
| 52 | H | H | 4-$CH_3$O | H | $CH_3$ | $CH_3$ | H | n-$C_3H_6$ | $C_2H_5$ | $C_2H_5$ | H-ma, öl; B, öl IR: ~3220, 1650, 1530 |
| 53 | H | H | 4-$CH_3$O | H | $CH_3$ | $CH_3$ | H | n-$C_3H_6$ | $C_2H_4$-O-$C_2H_4$ | | H-ma, öl; B, öl IR: ~3240, 1650, 1530 |
| 54 | H | H | 3,4-di-$CH_3$O | | $CH_3$ | $CH_3$ | H | $C_2H_4$ | -$(CH_2)_4$- | | H-ma, Fp. 172°C |
| 55 | H | H | 3,4-di-$CH_3$O | | $CH_3$ | $CH_3$ | H | n-$C_3H_6$ | $C_2H_5$ | $C_2H_5$ | H-ma, öl; B, öl IR: ~3220, 1650 |
| 56 | H | H | -O-$CH_2$-O- | | $CH_3$ | $CH_3$ | H | n-$C_3H_6$ | $C_2H_5$ | $C_2H_5$ | H-ma, öl; B, öl IR: ~3210, 1650, 1530 |
| 57 | $CH_3$ | H | H | H | $CH_3$ | $CH_3$ | H | $C_2H_4$ | -$(CH_2)_4$- | | H-ma, öl; B, Fp. 108°C |
| 58 | H | H | H | H | -n-$C_3H_7$ | H | H | $CH_2$-CHOH-$CH_2$ | $C_2H_5$ | $C_2H_5$ | H-ma, öl; B, öl IR: ~3240, 1658, 1530 |

B = Base, öl = ölig, Sch. = Schaumharz, H-ma = Hydrogenmaleinat, H-fu = Hydrogenfumarat, H2-Cit- = Dihydrogencytrat

| Beispiel Nr. | $R_1$ | $R_2$ | $R_3$ | $R_4$ | $R_5$ | $R_6$ | $R_7$ | Z | $R_8$ | $R_9$ | Bemerkungen |
|---|---|---|---|---|---|---|---|---|---|---|---|
| 59 | H | H | H | H | $CH_3$ | $CH_3$ | H | n-$C_4H_8$ | $C_2H_5$ | $C_2H_5$ | H-ma, öl; B, öl<br>IR: ~3210, 1655, 1530 |
| 60 | H | 1-$C_2H_5$ | H | H | $CH_3$ | $CH_3$ | H | n-$C_3H_6$ | $C_2H_5$ | $C_2H_5$ | H-ma, öl; B, SCH.<br>IR: 3430, 1665, 1535 |
| 61 | H | 1-$C_2H_5$ | H | H | $CH_3$ | $CH_3$ | H | $C_2H_4$ | $C_2H_5$ | $C_2H_5$ | H-ma, öl; B, öl<br>IR: ~3400, 1670 |
| 62 | H | $CH_3$<br>1-$CH_2$-CH<br>$CH_3$ | H | H | $CH_3$ | $CH_3$ | H | n-$C_3H_6$ | $C_2H_5$ | $C_2H_5$ | H-fu, öl; B, öl<br>IR: 3340, 1668, 1530 |
| 63 | H | H | 4-$NO_2$ | H | $CH_3$ | $CH_3$ | H | n-$C_3H_6$ | $C_2H_5$ | $C_2H_5$ | H-ma, öl; B, öl<br>IR: 3210, 1650, 1515 |
| 64 | H | 2-$CH_3$ | H | H | $CH_3$ | $CH_3$ | H | $CH_2$-CHOH-$CH_2$ | $C_2H_5$ | $C_2H_5$ | H-ma, öl; B, öl<br>IR: ~3340, ~1670, 1545 |
| 65 | H | 2-$CH_3$ | H | H | $CH_3$ | $CH_3$ | H | $CH_2$-CHOH-$CH_2$ | -$C_2H_4$-O-$C_2H_4$ | | B, öl<br>IR: ~3340, ~1665, 1545 |
| 66 | $CH_3$ | 1-$CH_3$ | H | H | $CH_3$ | $CH_3$ | H | n-$C_3H_6$ | $C_2H_5$ | $C_2H_5$ | H-ma, öl; B, öl<br>IR: 3350, 1670, 1520 |
| 67 | H | 2-$C_2H_5$ | H | H | Ch3 | $CH_3$ | H | n-$C_3H_6$ | $C_2H_5$ | $C_2H_5$ | H-fu, öl; B, öl<br>IR: 3315, 1665, 1545 |
| 68 | H | 2-n-$C_3H_7$ | H | H | $CH_3$ | $CH_3$ | H | n-$C_3H_6$ | $C_2H_5$ | $C_2H_5$ | H-ma, Fp. 95-100°C |
| 69 | H | $CH_3$<br>I<br>2-$CH_2$-CH<br>I<br>$CH_3$ | H | H | $CH_3$ | $CH_3$ | | n-$C_3H_6$ | $C_2H_5$ | $C_2H_5$ | H-ma, Fp. 105-115°C |
| 70 | H | H | H | H | $CH_3$ | $CH_3$ | H | $C_2H_4$ | -$(CH_2)_5$- | | B, öl<br>IR: 3200, 1658, 1520 |
| 71 | H | H | H | H | $CH_3$ | $CH_3$ | H | $C_2H_4$ | H | $CH_3$ | H-ma, Sch; B, öl<br>IR: ~3220, 1660, 1530 |
| 72 | H | H | H | H | n-$C_3H_7$ | H | H | n-$C_3H_6$ | $C_2H_5$ | $C_2H_5$ | H-ma, öl; B, öl<br>IR: ~3230, 1655, 1531 |
| 73 | H | H | H | H | $CH_3$ | $CH_3$ | H | n-$C_5H_{10}$ | $C_2H_5$ | $C_2H_5$ | H-ma, öl, B, öl<br>IR: 3210, 1655, 1532 |

B = Base, öl = ölig, Sch. = Schaumharz, H-ma = Hydrogenmaleinat, H-fu = Hydrogenfumarat, H2-Cit- = Dihydrogencytrat

*Beispiel I: Tabletten*

Man stellt Tabletten in folgender Zusammensetzung pro Tablette her:

| | |
|---|---|
| 3-[2-(3-Diäthylaminopropylaminocarbonyl)-propyl-2-oxy]-5-phenylpyrazol-hydrogenmaleinat | 15 mg |
| Maisstärke | 60 mg |
| Milchzucker | 140 mg |
| Gelatine (als 10%ige Lösung) | 6 mg |

Der Wirkstoff, die Maisstärke und der Milchzucker werden mit der 10%igen Gelatine-Lösung eingedickt. Die Paste wird zerkleinert und das entstandene Granulat wird auf ein geeignetes Blech gebracht und getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpresst.

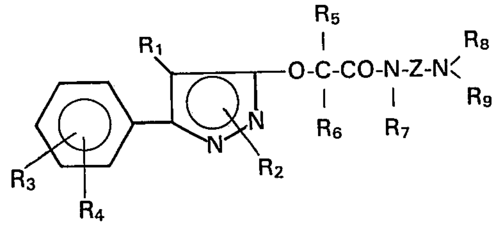

I

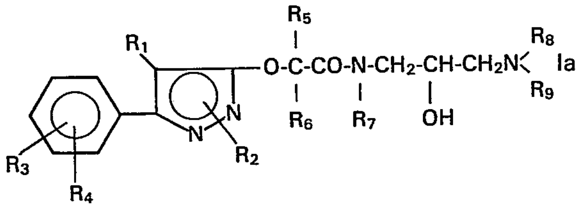

Ia

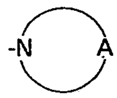

a

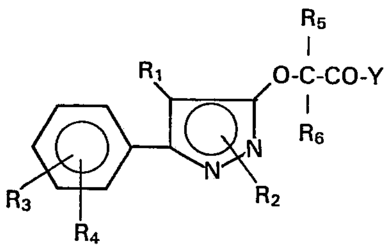

II

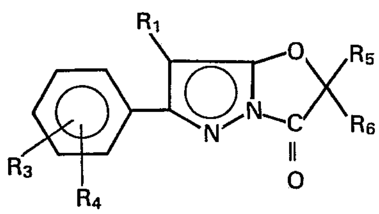

III

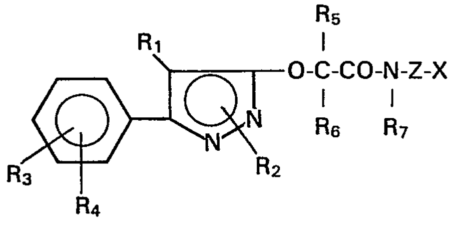

IV

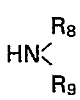

V

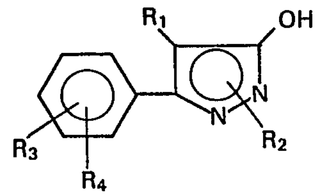

VI

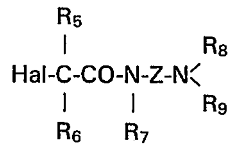

VII

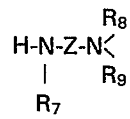

VIII

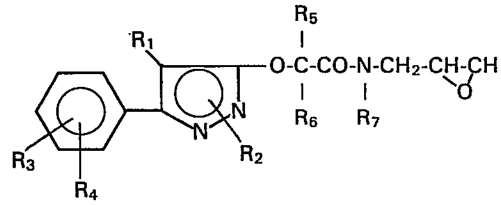

IX

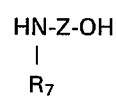

X

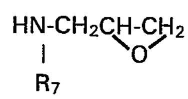

XI

$NH_2-NHR_2$

XII

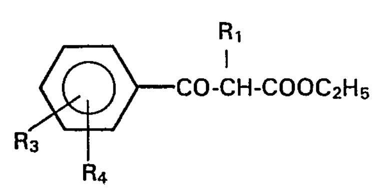

XIII

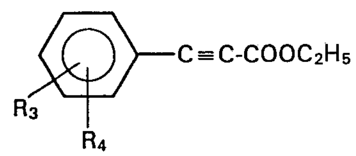

XIV

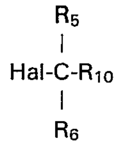

XV

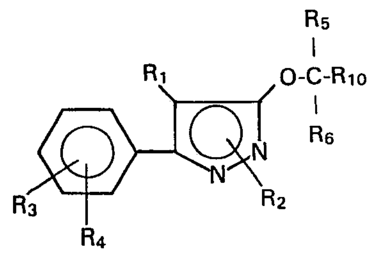

XVI

**Patentansprüche für die Vertragsstaaten:**
BE, CH, DE, FR, GB, IT, LU, NL, SE

1. 3-Aminocarbonylmethoxy-5-phenylpyrazol-Verbindungen der allgemeinen Formel I

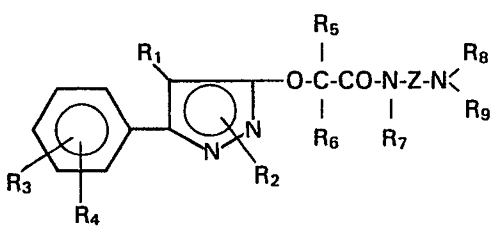

I

worin

$R_1$ Wasserstoff oder Alkyl mit 1-4 Kohlenstoffatomen bedeutet

$R_2$ in 1- oder 2-Stellung angeordnet ist und Wasserstoff oder Alkyl mit 1-4 Kohlenstoffatomen bedeutet

$R_3$ Wasserstoff, Halogen, Alkyl mit 1-4 Kohlenstoffatomen, Alkoxy mit 1-4 Kohlenstoffatomen bedeutet und

$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder

$R_3$ und $R_4$ an zwei benachbarte Kohlenstoffatome gebunden sind und gemeinsam eine Alkylendioxygruppe mit 1-2 Kohlenstoffatomen darstellen,

$R_5$ Wasserstoff oder Alkyl mit 1-4 Kohlenstoffatomen bedeutet und

$R_6$ Wasserstoff oder Methyl bedeutet oder

$R_5$ und $R_6$ gemeinsam eine Alkylenkette mit 3-5 Kohlenstoffatomen bilden,

$R_7$ Wasserstoff oder Alkyl mit 1-4 Kohlenstoffatomen bedeutet,

Z eine Alkylenkette mit 2-5 Kohlenstoffatomen oder die 2-Hydroxypropylenkette bedeutet,

$R_8$ Wasserstoff oder Alkyl mit 1-4 Kohlenstoffatomen bedeutet und

$R_9$ Wasserstoff oder Alkyl mit 1-4 Kohlenstoffatomen bedeutet oder

$R_8$ und $R_9$ gemeinsam mit dem Stickstoffatom, an das sie gebunden sind, eine heterocyclische Gruppe der allgemeinen Formel a

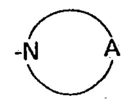

a

darstellen, worin A für eine gegebenenfalls durch 1-2 Methylgruppen substituierte Alkylenkette mit 4-5 Kohlenstoffatomen oder die $C_2H_4$-O-$C_2H_4$-kette steht, oder falls Z eine Alkylenkette ist, auch

$R_8$ Wasserstoff oder Alkyl mit 1-4 Kohlenstoffatomen bedeutet und

$R_9$ eine Alkylenkette ist und zusammen mit dem Stickstoffatom, an welches es gebunden ist, und dem diesem Stickstoffatom benachbarten Kohlenstoffatom der Alkylenkette Z einen Pyrrolidin- oder Piperidinring bildet,

sowie deren Säureadditionssalze.

2. 3-Aminocarbonylmethoxy-5-phenylpyrazol-Verbindungen gemäss Anspruch 1, worin

$R_3$ bis $R_9$ und Z die in Anspruch 1 angegebene Bedeutung besitzen,

$R_1$ Wasserstoff oder Alkyl mit 1-2 Kohlenstoffatomen bedeutet und

$R_2$ Wasserstoff oder Methyl bedeutet.

3. 3-Aminocarbonylmethoxy-5-phenylpyrazol-Verbindungen gemäss Anspruch 1, worin

$R_1$ bis $R_4$, $R_7$ bis $R_9$ die in Anspruch 1 angegebene Bedeutung besitzen und

$R_5$ und $R_6$ zusammen 3 Kohlenstoffatome besitzen.

4. 3-Aminocarbonylmethoxy-5-phenylpyrazol-Verbindungen gemäss Anspruch 3, worin

$R_5$ und $R_6$ je Methyl oder $R_5$ Alkyl mit 1-3 Kohlenstoffatomen und $R_6$ Wasserstoff bedeuten.

5. 3-Aminocarbonylmethoxy-5-phenylpyrazol-Verbindungen gemäss Anspruch 1, worin

$R_1$ bis $R_7$ und Z die in Anspruch 1 angegebene Bedeutung besitzen und

$R_8$ Wasserstoff oder Alkyl mit 1-2 Kohlenstoffatomen und

$R_9$ Wasserstoff oder Alkyl mit 1-2 Kohlenstoffatomen bedeuten oder

$R_8$ und $R_9$ gemeinsam mit dem Stickstoffatom, an welches sie gebunden sind, einen Pyrrolidin-, Piperidin- oder Morpholinring darstellen oder

$R_8$ Wasserstoff oder Methyl bedeutet und

$R_9$ zusammen mit dem Stickstoffatom und dem diesem benachbarten C-Atom der Alkylenkette Z einen Pyrrolidinring darstellt.

6. 3-Aminocarbonylmethoxy-5-phenylpyrazol-Verbindungen gemäss Anspruch 1, worin

$R_1$ Wasserstoff oder Alkyl mit 1-2 Kohlenstoffatomen bedeutet,

$R_2$ Wasserstoff oder Methyl bedeutet,

$R_3$ Wasserstoff, Halogen, Methoxy oder Methyl bedeutet,

$R_4$ Wasserstoff, Halogen, Methoxy oder Methyl bedeutet,

$R_5$ und $R_6$ je Methyl bedeuten,

$R_7$ Wasserstoff bedeutet,

Z eine Alkylenkette mit 2-4 Kohlenstoffatomen oder die 2-Hydroxypropylenkette darstellt, und

$R_8$ Wasserstoff oder Alkyl mit 1-2 Kohlenstoffatomen bedeutet und

$R_9$ Alkyl mit 1-2 Kohlenstoffatomen bedeutet oder

$R_8$ und $R_9$ gemeinsam mit dem Stickstoffatom, an welches sie gebunden sind, einen Pyrrolidin- oder Piperidinring bilden.

7. 3-Aminocarbonylmethoxy-5-phenylpyrazol-Verbindungen gemäss Anspruch 6, worin $R_1$ Wasserstoff oder Methyl bedeutet.

8. 3-Aminocarbonylmethoxy-5-phenylpyrazol-Verbindungen gemäss Anspruch 6, worin Z eine Alkylenkette mit 3 oder 4 Kohlenstoffatomen oder die 2-Hydroxypropylenkette darstellt.

9. 3-Aminocarbonylmethoxy-5-phenylpyrazol-Verbindungen gemäss Anspruch 1, worin $R_1$ eine Alkylgruppe mit 1-2 Kohlenstoffatomen bedeutet und Z eine Äthylenkette darstellt.

10. 3-Aminocarbonylmethoxy-5-phenylpyrazol-Verbindungen gemäss Anspruch 6, worin $R_8$ und $R_9$ je Alkyl mit 1-2 Kohlenstoffatomen bedeuten oder $R_8$ und $R_9$ gemeinsam mit dem Stickstoffatom, an welches sie gebunden sind, einen Pyrrolidin- oder Piperidinring bilden.

11. 3-[2-(3-Diäthylaminopropylaminocarbonyl)-propyl-2-oxy]-5-phenylpyrazol gemäss Anspruch 1 und dessen Säureadditionssalze.

12. 3-[2-(3-Diäthylamino-2-hydroxypropylaminocarbonyl)-propyl-2-oxy]-5-phenylpyrazol gemäss Anspruch 1 und dessen Säureadditionssalze.

13. 3-[1-(2-Pyrrolidinoäthylaminocarbonyl)-propyl-1-oxy]-5-(4-chlorphenyl)-pyrazol gemäss Anspruch 1 und dessen Säureadditionssalze.

14. Arzneimittel enthaltend eine pharmakologisch wirksame Menge einer 3-Aminocarbonylmethoxy-5-phenylpyrazol-Verbindung gemäss Anspruch 1 und übliche Hilfs- und/oder Trägerstoffe.

15. Verfahren zur Herstellung von Verbindungen der allgemeinen Formel I gemäss Anspruch 1

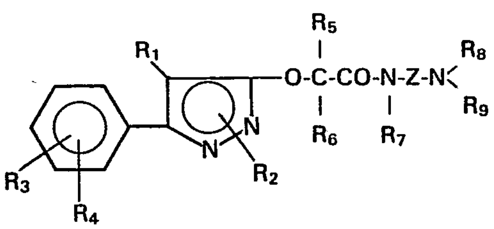
I

worin $R_1$, $R_2$, $R_3$, $R_4$, $R_5$, $R_6$, $R_7$, $R_8$, $R_9$ und Z die in Anspruch 1 angegebene Bedeutung besitzen, sowie deren Säureadditionssalzen, dadurch gekennzeichnet, dass man

a) Verbindungen der allgemeinen Formel II oder III

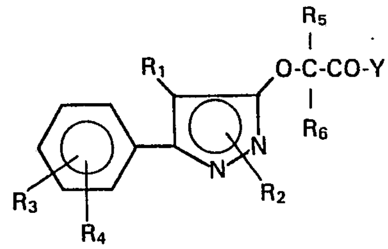
II

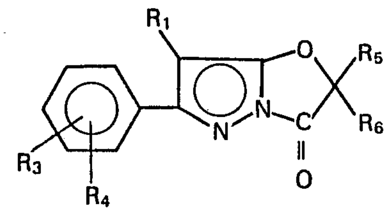
III

worin $R_1$, $R_2$, $R_3$, $R_4$, $R_5$ und $R_6$ obige Bedeutung besitzen und Y eine reaktive Gruppe bedeutet, mit Verbindungen der allgemeinen Formel VIII

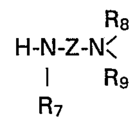
VIII

worin $R_7$, Z, $R_8$ und $R_9$ obige Bedeutungen besitzen, umsetzt, oder

b) Verbindungen der allgemeinen Formel IV

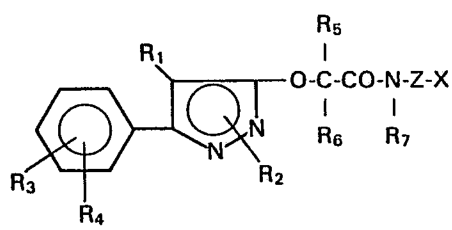
IV

worin $R_1$, $R_2$, $R_3$, $R_4$, $R_5$, $R_6$, $R_7$ und Z obige Bedeutung besitzen und X eine aminolytisch abspaltbare Gruppe bedeutet, mit Verbindungen der allgemeinen Formel V

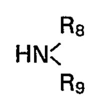
V

worin $R_8$ und $R_9$ obige Bedeutung besitzen, umsetzt, oder

c) Verbindungen der allgemeinen Formel VI

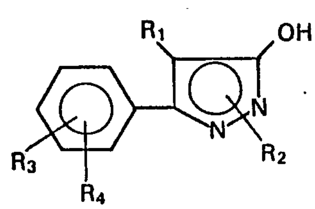
VI

worin $R_1$, $R_2$, $R_3$ und $R_4$ obige Bedeutung besitzen, und/oder die dazu tautomeren 5-Phenylpyrazolin-3-on-Verbindungen mit Verbindungen der allgemeinen Formel VII

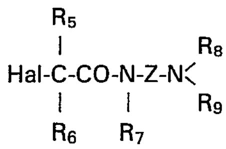
VII

worin $R_5$, $R_6$, $R_7$, Z, $R_8$ und $R_9$ obige Bedeutung besitzen und Hal Halogen bedeutet, umsetzt, oder

d) Zur Herstellung von Verbindungen der allgemeinen Formel Ia

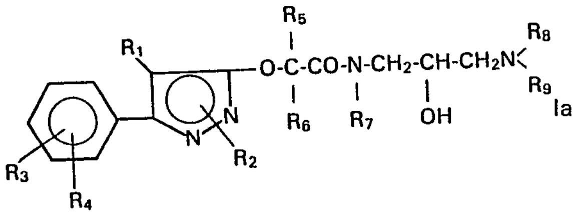
Ia

worin $R_1$, $R_2$, $R_3$, $R_4$, $R_5$, $R_6$, $R_7$, $R_8$ und $R_9$ obige Bedeutung besitzen, Verbindungen der allgemeinen Formel IX

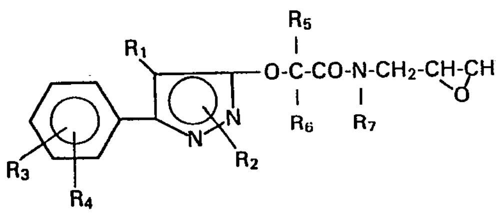
IX

worin $R_1$, $R_2$, $R_3$, $R_4$, $R_5$, $R_6$ und $R_7$ obige Bedeutung besitzen, umsetzt mit einer Verbindung der allgemeinen Formel V

und gegebenenfalls in Verbindungen der allgemeinen Formel I, worin $R_4$ Methoxy bedeutet, die Methoxygruppe zur Hydroxygruppe spaltet,

und gegebenenfalls freie Verbindungen der Formel I in ihre Säureadditionssalze überführt oder die Säureadditionssalze in die freien Verbindungen der Formel I überführt.

16. Verbindungen der allgemeinen Formel III

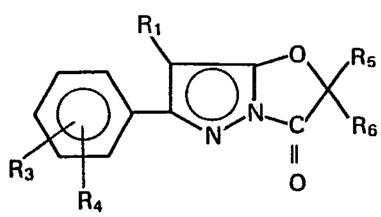
III

worin

$R_1$ Wasserstoff oder Alkyl mit 1-4 Kohlenstoffatomen bedeutet

$R_3$ Wasserstoff, Halogen, Alkyl mit 1-4 Kohlenstoffatomen oder Alkoxy mit 1-4 Kohlenstoffatomen bedeutet und

$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder

$R_3$ und $R_4$ an zwei benachbarte Kohlenstoffatome gebunden sind und gemeinsam eine Alkylendioxygruppe mit 1-2 Kohlenstoffatomen darstellen,

$R_5$ Wasserstoff oder Alkyl mit 1-4 Kohlenstoffatomen bedeutet und

$R_6$ Wasserstoff oder Methyl bedeutet oder

$R_5$ und $R_6$ gemeinsam eine Alkylenkette mit 3-5 Kohlenstoffatomen bilden.

17. Verbindungen der allgemeinen Formel IV

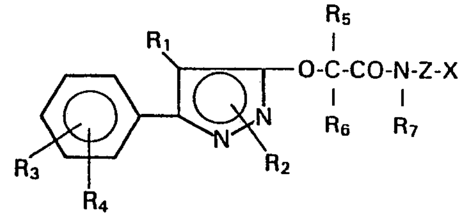
IV

worin

$R_1$ Wasserstoff oder Alkyl mit 1-4 Kohlenstoffatomen bedeutet,

$R_2$ in 1- oder 2-Stellung angeordnet ist und Wasserstoff oder Alkyl mit 1-4 Kohlenstoffatomen bedeutet,

$R_3$ Wasserstoff, Halogen, Alkyl mit 1-4 Kohlenstoffatomen oder Alkoxy mit 1-4 Kohlenstoffatomen bedeutet und

$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder

$R_3$ und $R_4$ an zwei benachbarte Kohlenstoffatome gebunden sind und gemeinsam eine Alkylendioxygruppe mit 1-2 Kohlenstoffatomen darstellen,

$R_5$ Wasserstoff oder Alkyl mit 1-4 Kohlenstoffatomen bedeutet und

$R_6$ Wasserstoff oder Methyl bedeutet oder

$R_5$ und $R_6$ gemeinsam eine Alkylenkette mit 3-5 Kohlenstoffatomen bilden,

$R_7$ Wasserstoff oder Alkyl mit 1-4 Kohlenstoffatomen bedeutet,

Z eine Alkylenkette mit 2-5 Kohlenstoffatomen oder die 2-Hydroxypropylenkette bedeutet, bedeutet und

X eine aminolytisch abspaltbare Gruppe bedeutet.

**Patentansprüche für den Vertragsstaat:** AT

1. Verfahren zur Hertellung von Verbindungen der allgemeinen Formel I

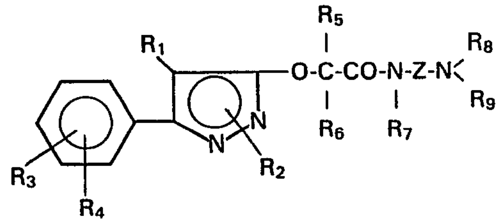
I

worin
$R_1$ Wasserstoff oder Alkyl mit 1-4 Kohlenstoffatomen bedeutet,
$R_2$ in 1- oder 2-Stellung angeordnet ist und Wasserstoff oder Alkyl mit 1-4 Kohlenstoffatomen bedeutet,
$R_3$ Wasserstoff, Halogen, Alkyl mit 1-4 Kohlenstoffatomen oder Alkoxy mit 1-4 Kohlenstoffatomen bedeutet und
$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder
$R_3$ und $R_4$ an zwei benachbarte Kohlenstoffatome gebunden sind und gemeinsam eine Alkylendioxygruppe mit 1-2 Kohlenstoffatomen darstellen,
$R_5$ Wasserstoff oder Alkyl mit 1-4 Kohlenstoffatomen bedeutet und
$R_6$ Wasserstoff oder Methyl bedeutet oder
$R_5$ und $R_6$ gemeinsam eine Alkylenkette mit 3-5 Kohlenstoffatomen bilden,
$R_7$ Wasserstoff oder Alkyl mit 1-4 Kohlenstoffatomen bedeutet,
Z eine Alkylenkette mit 2-5 Kohlenstoffatomen oder die 2-Hydroxypropylenkette bedeutet,
$R_8$ Wasserstoff oder Alkyl mit 1-4 Kohlenstoffatomen bedeutet und
$R_9$ Wasserstoff oder Alkyl mit 1-4 Kohlenstoffatomen bedeutet oder
$R_8$ und $R_9$ gemeinsam mit dem Stickstoffatom, an das sie gebunden sind, eine heterocyclische Gruppe der allgemeinen Formel a

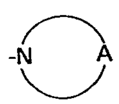

a

darstellen, worin A für eine gegebenenfalls durch 1-2 Methylgruppen substituierte Alkylenkette mit 4-5 Kohlenstoffatomen oder die $C_2H_4$-O-$C_2H_4$-kette steht, oder falls Z eine Alkylenkette ist, auch
$R_8$ Wasserstoff oder Alkyl mit 1-4 Kohlenstoffatomen bedeutet und
$R_9$ eine Alkylenkette ist und zusammen mit dem Stickstoffatom, an welches es gebunden ist, und dem diesem Stickstoffatom benachbarten Kohlenstoffatom der Alkylenkette Z einen Pyrrolidin- oder Piperidinring bildet,
sowie deren Säureadditionssalzen, dadurch gekennzeichnet, dass man

a) Verbindungen der allgemeinen Formel II oder III

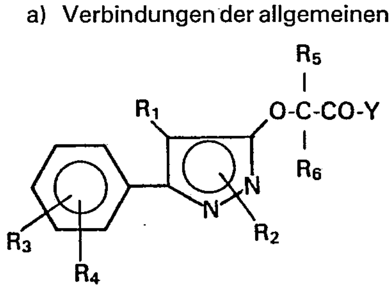

II

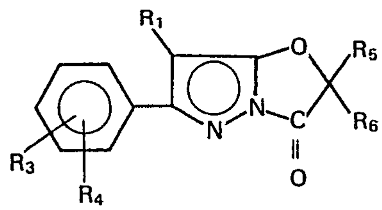

III

worin $R_1$, $R_2$, $R_3$, $R_4$, $R_5$ und $R_6$ obige Bedeutung besitzen und Y eine reaktive Gruppe bedeutet, mit Verbindungen der allgemeinen Formel VIII

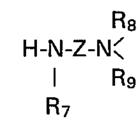

VIII

worin $R_7$, Z, $R_8$ und $R_9$ obige Bedeutungen besitzen, umsetzt, oder

b) Verbindungen der allgemeinen Formel IV

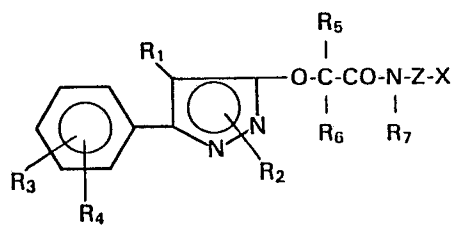

IV

worin $R_1$, $R_2$, $R_3$, $R_4$, $R_5$, $R_6$, $R_7$ und Z obige Bedeutung besitzen und X eine aminolytisch abspaltbare Gruppe bedeutet, mit Verbindungen der allgemeinen Formel V

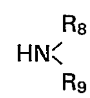

V

worin $R_8$ und $R_9$ obige Bedeutung besitzen, umsetzt, oder

c) Verbindungen der allgemeinen Formel VI

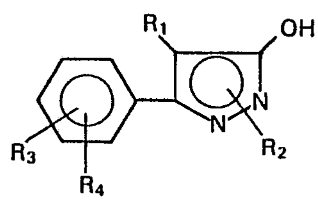

VI

worin $R_1$, $R_2$, $R_3$ und $R_4$ obige Bedeutung besitzen, und/oder die dazu tautomeren 5-Phenylpyrazlin-3-on-Verbindungen mit Verbindungen der allgemeinen Formel VII

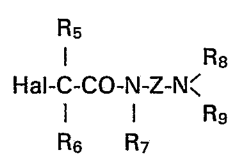

VII

worin $R_5$, $R_6$, $R_7$, Z, $R_8$ und $R_9$ obige Bedeutung besitzen und Hal Halogen bedeutet, umsetzt, oder

d) zur Herstellung von Verbindungen der allgemeinen Formel Ia

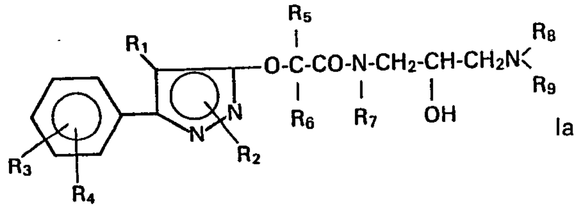

Ia

worin $R_1$, $R_2$, $R_3$, $R_4$, $R_5$, $R_6$, $R_7$, $R_8$ und $R_9$ obige Bedeutung besitzen, Verbindungen der allgemeinen Formel IX

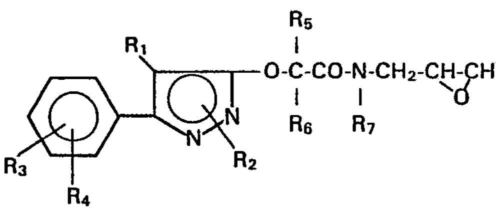

IX

worin $R_1$, $R_2$, $R_3$, $R_4$, $R_5$, $R_6$ und $R_7$ obige Bedeutung besitzen, umsetzt mit einer Verbindung der allgemeinen Formel V

und gegebenenfalls in Verbindungen der allgemeinen Formel I, worin $R_4$ Methoxy bedeutet, die Methoxygruppe zur Hydroxygruppe spaltet,

und gegebenenfalls freie Verbindungen der Formel I in ihre Säureadditionssalze überführt oder die Säureadditionssalze in die freien Verbindungen der Formel I überführt.

2. Verfahren nach Anspruch 1, dadurch gekennzeichnet, dass man 3-Aminocarbonylmethoxy-5-phenylpyrazol-Verbindungen gemäss Anspruch 1 herstellt, worin

$R_3$ bis $R_9$ und Z die in Anspruch 1 angegebene Bedeutung besitzen,

$R_1$ Wasserstoff oder Alkyl mit 1-2 Kohlenstoffatomen bedeutet und

$R_2$ Wasserstoff oder Methyl bedeutet.

3. Verfahren nach Anspruch 1, dadurch gekennzeichnet, dass man 3-Aminocarbonylmethoxy-5-phenylpyrazol-Verbindungen gemäss Anspruch 1 herstellt, worin

$R_1$ bis $R_4$, $R_7$ bis $R_9$ die in Anspruch 1 angegebene Bedeutung besitzen und

$R_5$ und $R_6$ zusammen 3 Kohlenstoffatome besitzen.

4. Verfahren nach Anspruch 3, dadurch gekennzeichnet, dass man 3-Aminocarbonylmethoxy-5-phenylpyrazol-Verbindungen gemäss Anspruch 3 herstellt, worin

$R_5$ und $R_6$ je Methyl oder $R_5$ Alkyl mit 1-3 Kohlenstoffatomen und $R_6$ Wasserstoff bedeuten.

5. Verfahren nach Anspruch 1, dadurch gekennzeichnet, dass man 3-Aminocarbonylmethoxy-5-phenylpyrazol-Verbindungen gemäss Anspruch 1 herstellt, worin

$R_1$ bis $R_7$ und Z die in Anspruch 1 angegebene Bedeutung besitzen und

$R_8$ Wasserstoff oder Alkyl mit 1-2 Kohlenstoffatomen und

$R_9$ Wasserstoff oder Alkyl mit 1-2 Kohlenstoffatomen bedeuten oder

$R_8$ und $R_9$ gemeinsam mit dem Stickstoffatom, an welches sie gebunden sind, einen Pyrrolidin-, Piperidin- oder Morpholinring darstellen oder

$R_8$ Wasserstoff oder Methyl bedeutet und

$R_9$ zusammen mit dem Stickstoffatom und dem diesem benachbarten C-Atom der Alkylenkette Z einen Pyrrolidinring darstellt.

6. Verfahren nach Anspruch 6, dadurch gekennzeichnet, dass man 3-Aminocarbonylmethoxy-5-phenylpyrazol-Verbindungen gemäss Anspruch 1 herstellt, worin

$R_1$ Wasserstoff oder Alkyl mit 1-2 Kohlenstoffatomen bedeutet,

$R_2$ Wasserstoff oder Methyl bedeutet,

$R_3$ Wasserstoff, Halogen, Methoxy oder Methyl bedeutet,

$R_4$ Wasserstoff, Halogen, Methoxy oder Methyl bedeutet,

$R_5$ und $R_6$ je Methyl bedeuten,

$R_7$ Wasserstoff bedeutet,

Z eine Alkylenkette mit 2-4 Kohlenstoffatomen oder die 2-Hydroxypropylenkette darstellt, und

$R_8$ Wasserstoff oder Alkyl mit 1-2 Kohlenstoffatomen bedeutet und

$R_9$ Alkyl mit 1-2 Kohlenstoffatomen bedeutet oder

$R_8$ und $R_9$ gemeinsam mit dem Stickstoffatom, an welches sie gebunden sind, einen Pyrrolidin- oder Piperidinring bilden.

7. Verfahren nach Anspruch 6, dadurch gekennzeichnet, dass man 3-Aminocarbonylmethoxy-5-phenylpyrazol-Verbindungen gemäss Anspruch 6 herstellt, worin $R_1$ Wasserstoff oder Methyl bedeutet.

8. Verfahren nach Anspruch 6, dadurch gekennzeichnet, dass man 3-Aminocarbonylmethoxy-5-phenylpyrazol-Verbindungen gemäss Anspruch 6 herstellt, worin Z eine Alkylenkette mit 3 oder 4 Kohlenstoffatomen oder die 2-Hydroxypropylenkette darstellt.

9. Verfahren nach Anspruch 6, dadurch gekennzeichnet, dass man 3-Aminocarbonylmethoxy-5-phenylpyrazol-Verbindungen gemäss Anspruch 6 herstellt, worin $R_1$ eine Alkylgruppe mit 1-2 Kohlenstoffatomen bedeutet und Z eine Äthylenkette darstellt.

10. Verfahren nach Anspruch 6, dadurch gekennzeichnet, dass man 3-Aminocarbonylmethoxy-5-phenylpyrazol-Verbindungen gemäss Anspruch 6 herstellt, worin $R_8$ und $R_9$ je Alkyl mit 1-2 Kohlenstoffatomen bedeuten oder

$R_8$ und $R_9$ gemeinsam mit dem Stickstoffatom, an welches sie gebunden sind, einen Pyrrolidin- oder Piperidinring bilden.

11. Verfahren nach Anspruch 1, dadurch gekennzeichnet, dass man als Verbindung der Formel I 3-[2-(3-Diäthylaminopropylaminocarbonyl)-propyl-2-oxy]-5-phenylpyrazol und dessen Säureadditionssalze herstellt.

12. Verfahren nach Anspruch 1, dadurch gekennzeichnet, dass man als Verbindung der Formel I 3-[2-(3-Diäthylamino-2-hydroxypropylaminocarbonyl)-propyl-2-oxy]-5-phenylpyrazol und dessen Säureadditionssalze herstellt.

13. Verfahren nach Anspruch 1, dadurch gekennzeichnet, dass man als Verbindung der Formel I 3-[1-(2-Pyrrolidinoäthylaminocarbonyl)-propyl-1-oxy]-5-(4-chlorphenyl)-pyrazol und dessen Säureadditionssalze herstellt.

14. Verfahren zur Herstellung von Verbindungen der allgemeinen Formel III

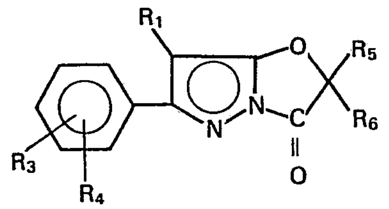

III

worin

$R_1$ Wasserstoff oder Alkyl mit 1-4 Kohlenstoffatomen bedeutet,

$R_3$ Wasserstoff, Halogen, Alkyl mit 1-4 Kohlenstoffatomen oder Alkoxy mit 1-4 Kohlenstoffatomen bedeutet und

$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder

$R_3$ und $R_4$ an zwei benachbarte Kohlenstoffatome gebunden sind und gemeinsam eine Alkylendioxygruppe mit 1-2 Kohlenstoffatomen darstellen,

$R_5$ Wasserstoff oder Alkyl mit 1-4 Kohlenstoffatomen bedeutet und

$R_6$ Wasserstoff oder Methyl bedeutet oder

$R_5$ und $R_6$ gemeinsam eine Alkylenkette mit 3-5 Kohlenstoffatomen bilden,

dadurch gekennzeichnet, dass man Verbindungen der allgemeinen Formel II'

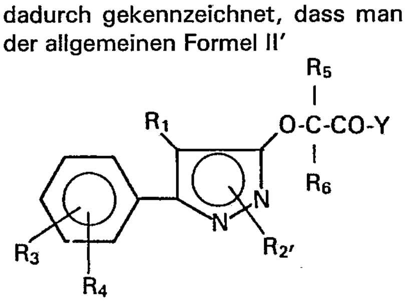

II'

worin $R_1$, $R_3$, $R_4$, $R_5$ und $R_6$ obige Bedeutung besitzen, $R_2'$ Wasserstoff bedeutet und Y eine reaktive Gruppe bedeutet, kondensiert.

15. Verfahren zur Herstellung von Verbindungen der allgemeinen Formel IV

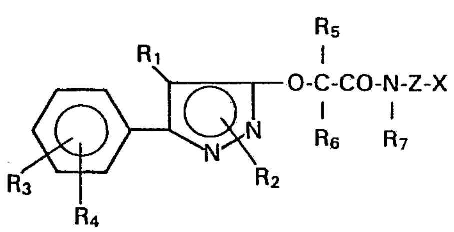

IV

worin

$R_1$ Wasserstoff oder Alkyl mit 1-4 Kohlenstoffatomen bedeutet,

$R_2$ in 1- oder 2-Stellung angeordnet ist und Wasserstoff oder Alkyl mit 1-4 Kohlenstoffatomen bedeutet,

$R_3$ Wasserstoff, Halogen, Alkyl mit 1-4 Kohlenstoffatomen oder Alkoxy mit 1-4 Kohlenstoffatomen bedeutet und

$R_4$ Wasserstoff, Halogen, Alkyl mit 1-4 Kohlenstoffatomen, Alkoxy mit 1-4 Kohlenstoffatomen oder, falls $R_3$ Wasserstoff ist, auch Trifluormethyl, Nitro oder Hydroxy bedeutet oder

$R_3$ und $R_4$ an zwei benachbarte Kohlenstoffatome gebunden sind und gemeinsam eine Alkylendioxygruppe mit 1-2 Kohlenstoffatomen darstellen,

$R_5$ Wasserstoff oder Alkyl mit 1-4 Kohlenstoffatomen bedeutet und

$R_6$ Wasserstoff oder Methyl bedeutet oder

$R_5$ und $R_6$ gemeinsam eine Alkylenkette mit 3-5 Kohlenstoffatomen bilden,

$R_7$ Wasserstoff oder Alkyl mit 1-4 Kohlenstoffatomen bedeutet,

Z eine Alkylenkette mit 2-5 Kohlenstoffatomen oder die 2-Hydroxypropylenkette bedeutet, und

X eine aminolytisch abspaltbare Gruppe bedeutet,

dadurch gekennzeichnet, dass man Verbindungen der allgemeinen Formel II

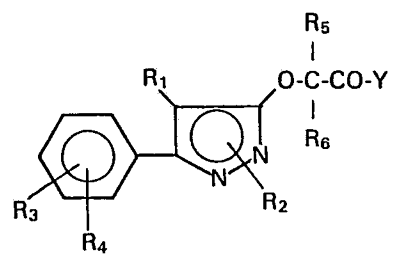

II

worin $R_1$, $R_2$, $R_3$, $R_4$, $R_5$ und $R_6$ obige Bedeutung besitzen und Y eine reaktive Gruppe bedeutet mit Verbindungen der allgemeinen Formel X

HN-Z-OH  X
|
$R_7$

worin $R_7$ und Z obige Bedeutung besitzen, umsetzt und anschliessend in den erhaltenen Reaktionsprodukten die endständige Hydxroxygruppe der 3-Seitenkette in eine aminolytisch abspaltbare Gruppe X überführt.

**Claims for the contracting States:**
BE, CH, DE, FR, GB, IT, LU, NL, SE

1. 3-Aminocarbonylmethoxy-5-phenylpyrazole compounds of the general Formula I

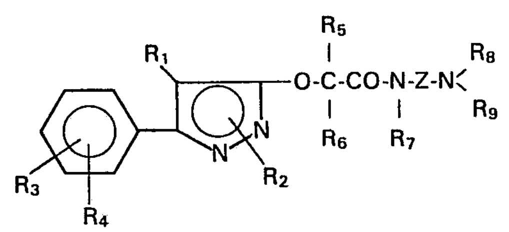

I

in which

$R_1$ denotes hydrogen or alkyl with 1-4 carbon atoms,

$R_2$ is arranged in position 1 or 2 and denotes hydrogen or alkyl with 1-4 carbon atoms,

$R_3$ denotes hydrogen, halogen, alkyl with 1-4 carbon atoms or alkoxy with 1-4 carbon atoms and

$R_4$ denotes hydrogen, halogen alkyl with 1-4 carbon atoms, alkoxy with 1-4 carbon atoms or, if $R_3$ is hydrogen, also denotes trifluormethyl, nitro or hydroxy or

$R_3$ and $R_4$ are linked to two adjacent carbon atoms and together represent an alkylene dioxy group with 1-2 carbon atoms,

$R_5$ denotes hydrogen or alkyl with 1-4 carbon atoms and

$R_6$ denotes hydrogen or methyl or

$R_5$ and $R_6$ together form an alkylene chain with 3-5 carbon atoms,

$R_7$ denotes hydrogen or alkyl with 1-4 carbon atoms,

Z denotes an alkylene chain with 2-5 carbon atoms or denotes the 2-hydroxypropylene chain,

$R_8$ denotes hydrogen or alkyl with 1-4 carbon atoms and

$R_9$ denotes hydrogen or alkyl with 1-4 carbon atoms or

$R_8$ and $R_9$ together with the nitrogen atom to which they are linked, represent a heterocyclic group of the general Formula a

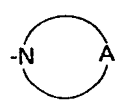

a

in which A stands for an alkylene chain, which optionally may be substituted by 1-2 methyl groups, with 4-5 carbon atoms or stands for the $C_2H_4$-O-$C_2H_4$- chain, or if Z is an alkylene chain, also

$R_8$ denotes hydrogen or alkyl with 1-4 carbon atoms and

$R_9$ is an alkylene chain and together with the nitrogen atom to which it is linked, and the carbon atom of the alkylene chain Z which is adjacent to this nitrogen atom, forms a pyrrolidine- or piperidine ring,

and their acid addition salts.

2. 3-Aminocarbonylmethoxy-5-phenylpyrazole compounds according to claim 1, in which

$R_3$ to $R_9$ and Z have the meaning given in claim 1,

$R_1$ denotes hydrogen or alkyl with 1-2 carbon atoms and

$R_2$ denotes hydrogen or methyl.

3. 3-Aminocarbonymethoxy-5-phenylpyrazole compounds according to claim 1, in which

$R_1$ to $R_4$, $R_7$ to $R_9$ and Z have the meaning given in claim 1 and

$R_5$ and $R_6$ together have 3 carbon atoms.

4. 3-Aminocarbonylmethoxy-5-phenylpyrazole compounds according to claim 3, in which

$R_5$ and $R_6$ each denote methyl or $R_5$ denotes alkyl with 1-3 carbon atoms and $R_6$ denotes hydrogen.

5. 3-Aminocarbonylmethoxy-5-phenylpyrazole compounds according to claim 1, in which

$R_1$ and $R_7$ and Z have the meaning given in claim 1 and

$R_8$ denotes hydrogen or alkyl with 1-2 carbon atoms and

$R_9$ denotes hydrogen or alkyl with 1-2 carbon atoms or

$R_8$ and $R_9$ together with the nitrogen atom to which they are linked, represent a pyrrolidine-, piperdine- or morpholine ring or

$R_8$ denotes hydrogen or methyl and

$R_9$ together with the nitrogen atom and the C-atom of the alkylene chain Z adjacent thereto represents a pyrrolidine ring.

6. 3-Aminocarbonylmethoxy-5-phenylpyrazole compounds according to claim 1, in which

$R_1$ denotes hydrogen or alkyl with 1-2 carbon atoms,

$R_2$ denotes hydrogen or methyl,

$R_3$ denotes hydrogen, halogen, methoxy or methyl,

$R_4$ denotes hydrogen, halogen, methoxy or methyl,

$R_5$ and $R_6$ each denote methyl,

$R_7$ denotes hydrogen,

Z represents an alkylene chain with 2-4 carbon atoms or the 2-hydroxypropylene chain, and

$R_8$ denotes hydrogen or alkyl with 1-2 carbon atoms and

$R_9$ denotes alkyl with 1-2 carbon atoms or

$R_8$ and $R_9$, together with the nitrogen atom to which they are linked, form a pyrrolidine- or piperidine ring.

7. 3-Aminocarbonylmethoxy-5-phenylpyrazole compounds according to claim 6, in which $R_1$ denotes hydrogen or methyl.

8. 3-Aminocarbonylmethoxy-5-phenylpyrazole compounds according to claim 6, in which Z represents an alkylene chain with 3 or 4 carbon atoms or the 2-hydroxypropylene chain.

9. 3-Aminocarbonylmethoxy-5-phenylpyrazole compounds to claim 6, in which $R_1$ denotes an alkyl group with 1-2 carbon atoms and Z represents an ethylene chain.

10. 3-Aminocarbonylmethoxy-5-phenylpyrazole compounds according to claim 6, in which $R_8$ and $R_9$ each denote alkyl with 1-2 carbon atoms or

$R_8$ and $R_9$ together with the nitrogen atom to which they are linked form a pyrrolidine- or piperidine ring.

11. 3-[2-(3-Diethylaminopropylaminocarbonyl)-propyl-2-oxy]-5-phenylpyrazole according to claim 1 and its acid addition salts.

12. 3-[2-(3-Diethylamino-2-hydroxypropylaminocarbonyl)-propyl-2-oxy]-5-phenylpyrazole according to claim 1 and its acid addition salts.

13. 3-[2-(3-Pyrrolidinoethylaminocarbonyl)-propyl-1-oxy]-5-(4-chlorphenyl)-pyrazole according to claim 1 and its acid addition salts.

14. Medicament containing a pharmacologically effective quantity of a 3-aminocarbonylmethoxy-5-phenylpyrazole compound according to claim 1 and conventional adjuvant- and/or carrier substances.

15. Method for the preparation of compounds of the general Formula I according to claim 1

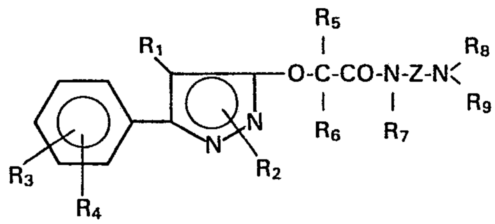

I

in which $R_1$, $R_2$, $R_3$, $R_4$, $R_5$, $R_6$, $R_7$, $R_8$, $R_9$ and Z have the meaning given in claim 1,
and their acid addition salts, characterized in that

a) compounds of the general Formule II or III

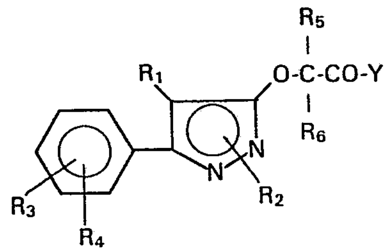

II

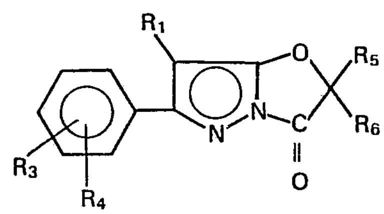

III

in which $R_1$, $R_2$, $R_3$, $R_4$, $R_5$ and $R_6$ have the above meaning and Y denotes a reactive group, are reacted with compounds of the general Formula VIII

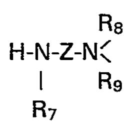

VIII

in which $R_7$, Z, $R_8$ and $R_9$ have the above meanings, or

b) compounds of the general Formula IV

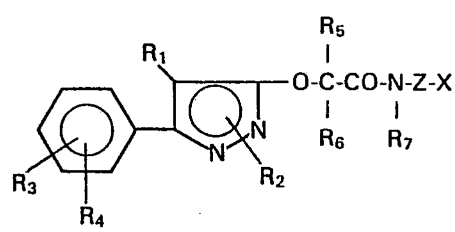

IV

in which $R_1$, $R_2$, $R_3$, $R_4$, $R_5$, $R_6$, $R_7$ and Z have the above meaning and X denotes an aminolytically separable group, are reacted with compounds of the general Formula V

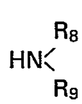

V

in which $R_8$ and $R_9$ have the above meaning, or

c) compounds of the general Formula VI

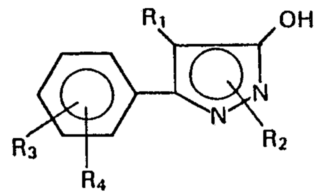

VI

in which $R_1$, $R_2$, $R_3$ and $R_4$ have the above meaning, and/or the 5-phenylpyrazoline-3-one compounds tautomeric thereto are reacted with compounds of the general Formula VII

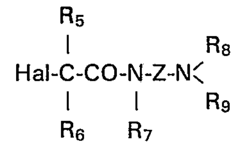

VII

in which $R_5$, $R_6$, $R_7$, Z, $R_8$ and $R_9$ have the above meaning and Hal denotes halogen, or

d) for the preparation of compounds of the general Formula Ia

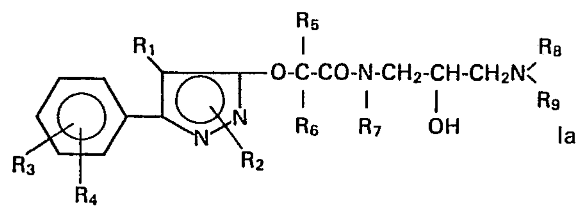

Ia

in which $R_1$, $R_2$, $R_3$, $R_4$, $R_5$, $R_6$, $R_7$, $R_8$ and $R_9$ have the above meaning, compounds of the general Formula IX

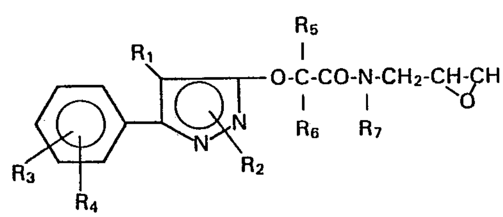

IX

in which $R_1$, $R_2$, $R_3$, $R_4$, $R_5$, $R_6$ and $R_7$ have the above meaning, are reacted with a compound of the general Formula V
and optionally in compounds of the general Formula I in which $R_4$ denotes methoxy, the methoxy group is split to the hydroxy group, and optionally free compounds of Formula I are converted into the their acid addition salts or the acid addition salts are converted into the free compounds of Formula I.

16. Compounds of the general Formula III

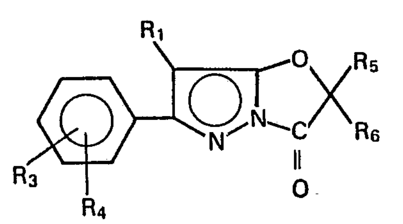

III

in which

$R_1$ denotes hydrogen or alkyl with 1-4 carbon atoms,

$R_3$ denotes hydrogen, halogen, alkyl with 1-4 carbon atoms or alkoxy with 1-4 carbon atoms and

$R_4$ denotes hydrogen, halogen alkyl with 1-4 carbon atoms, alkoxy with 1-4 carbon atoms or, if $R_3$ is hydrogen, also denotes trifluormethyl, nitro or hydroxy or

$R_3$ and $R_4$ are linked to two adjacent carbon atoms and together represent an alkylene dioxy group with 1-2 carbon atoms,

$R_5$ denotes hydrogen or alkyl with 1-4 carbon atoms and

$R_6$ denotes hydrogen or methyl or

$R_5$ and $R_6$ together form an alkylene chain with 3-5 carbon atoms.

17. Compounds of the general Formula IV

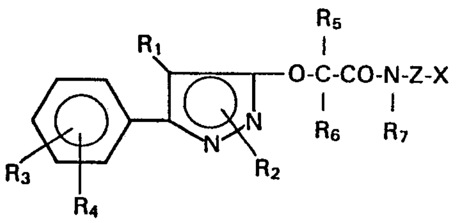

IV

in which

$R_1$ denotes hydrogen or alkyl with 1-4 carbon atoms,

$R_2$ is arranged in position 1 or 2 and denotes hydrogen or alkyl with 1-4 carbon atoms,

$R_3$ denotes hydrogen, halogen, alkyl with 1-4 carbon atoms or alkoxy with 1-4 carbon atoms and

$R_4$ denotes hydrogen, halogen alkyl with 1-4 carbon atoms, alkoxy with 1-4 carbon atoms or, if $R_3$ is hydrogen, also denotes trifluormethyl, nitro or hydroxy or

$R_3$ and $R_4$ are linked to two adjacent carbon atoms and together represent an alkylene dioxy group with 1-2 carbon atoms,

$R_5$ denotes hydrogen or alkyl with 1-4 carbon atoms and

$R_6$ denotes hydrogen or methyl or

$R_5$ and $R_6$ together form an alkylene chain with 3-5 carbon atoms,

$R_7$ denotes hydrogen or alkyl with 1-4 carbon atoms,

Z denotes an alkylene chain with 2-5 carbon atoms or denotes the 2-hydroxypropylene chain, and

X denotes an aminolytically separable group.

**Claims for the contracting State:** AT

1. Method for the preparation of compounds of the general Formula I

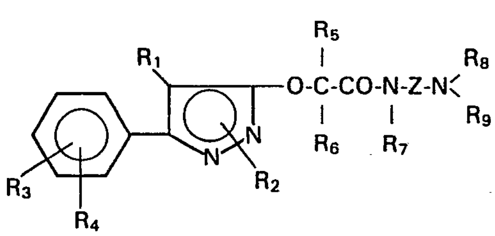

I

in which

$R_1$ denotes hydrogen or alkyl with 1-4 carbon atoms,

$R_2$ is arranged in position 1 or 2 and denotes hydrogen or alkyl with 1-4 carbon atoms,

$R_3$ denotes hydrogen, halogen, alkyl with 1-4 carbon atoms or alkoxy with 1-4 carbon atoms and

$R_4$ denotes hydrogen, halogen alkyl with 1-4 carbon atoms, alkoxy with 1-4 carbon atoms or, if $R_3$ is hydrogen, also denotes trifluormethyl, nitro or hydroxy or

$R_3$ and $R_4$ are linked to two adjacent carbon atoms and together represent an alkylene dioxy group with 1-2 carbon atoms,

$R_5$ denotes hydrogen or alkyl with 1-4 carbon atoms and

$R_6$ denotes hydrogen or methyl or

$R_5$ and $R_6$ together form an alkylene chain with 3-5 carbon atoms,

$R_7$ denotes hydrogen or alkyl with 1-4 carbon atoms,

Z denotes an alkylene chain with 2-5 carbon atoms or denotes the 2-hydroxypropylene chain,

$R_8$ denotes hydrogen or alkyl with 1-4 carbon atoms and

$R_9$ denotes hydrogen or alkyl with 1-4 carbon atoms or

$R_8$ and $R_9$ together with the nitrogen atom to which they are linked represent a heterocyclic group of the general Formula a

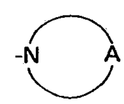

a

in which A stands for an alkylene chain, which optionally may be substituted by 1-2 methyl groups, with 4-5 carbon atoms or stands for the $C_2H_4$-O-$C_2H_4$- chain, or if Z is an alkylene chain, also

$R_8$ denotes hydrogen or alkyl with 1-4 carbon atoms and

$R_9$ is an alkylene chain and together with the nitrogen atom to which it is linked, and the carbon atoms of the alkylene chain Z which is adjacent to this nitrogen atom, forms a pyrrolidine- or piperidine ring,

and their acid addition salts, characterized in that

a) compounds of the general Formula II or III

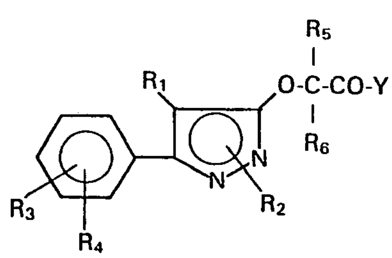

II

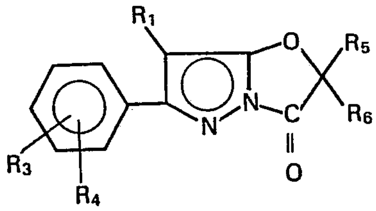

III

in which $R_1$, $R_2$, $R_3$, $R_4$, $R_5$ and $R_6$ have the above meaning and Y denotes a reactive group, are reacted with compounds of the general Formula VIII

```
        R8
H-N-Z-N<              VIII
  |     R9
  R7
```

in which $R_7$, Z, $R_8$ and $R_9$ have the above meanings, or

b) compounds of the general Formula IV

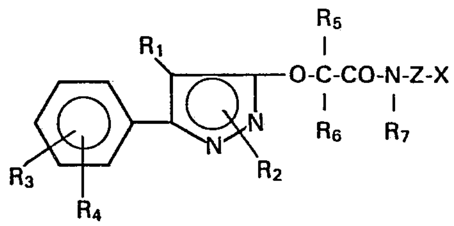

IV

in which $R_1$, $R_2$, $R_3$, $R_4$, $R_5$, $R_6$, $R_7$ and Z have the above meaning and X denotes an aminolytically separable group, are reacted with compounds of the general Formula V

```
   R8
HN<          V
   R9
```

in which $R_8$ and $R_9$ have the above meaning, or

c) compounds of the general Formula VI

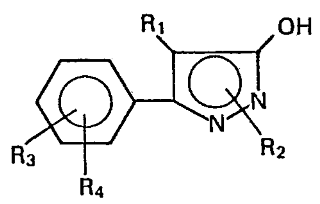

VI

in which $R_1$, $R_2$, $R_3$ and $R_4$ have the above meaning, and/or the 5-phenylpyrazoline-3-one compounds tautomeric thereto are reacted with compounds of the general Formula VII

```
    R5
    |           R8
Hal-C-CO-N-Z-N<              VII
    |    |      R9
    R6   R7
```

in which $R_5$, $R_6$, $R_7$, Z, $R_8$ and $R_9$ have the above meaning and Hal denotes halogen, or

d) for the preparation of compounds of the general Formula Ia

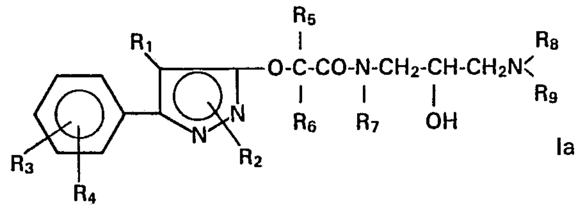

Ia

in which $R_1$, $R_2$, $R_3$, $R_4$, $R_5$, $R_6$, $R_7$, $R_8$ and $R_9$ have the above meaning, compounds of the general Formula IX

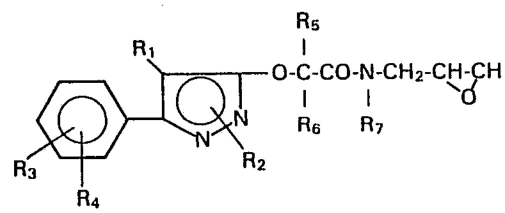

IX

in which $R_1$, $R_2$, $R_3$, $R_4$, $R_5$, $R_6$ and $R_7$ have the above meaning, are reacted with a compound of the general Formula V and optionally in compounds of the general Formula I in which $R_4$ denotes methoxy, the methoxy group is split to the hydroxy group, and optionally free compounds of Formula I are converted into the their acid addition salts or the acid addition salts are converted into the free compounds of Formula I.

2. Method according to claim 1, characterized in that 3-aminocarbonylmethoxy-5-phenylpyrazole compounds according to claim 1 are produced, in which

$R_3$ to $R_9$ and Z have the meaning given in claim 1,

$R_1$ denotes hydrogen or alkyl with 1-2 carbon atoms and

$R_2$ denotes hydrogen or methyl.

3. Method according to claim 1, characterized in that 3-aminocarbonymethoxy-5-phenylpyrazole compounds according to claim 1 are produced, in which

$R_1$ to $R_4$, $R_7$ to $R_9$ and Z have the meaning given in claim 1 and

$R_5$ and $R_6$ together have 3 carbon atoms.

4. Method according to claim 3, characterized in that 3-aminocarbonylmethoxy-5-phenylpyrazole compounds according to claim 3 are produced, in which

$R_5$ and $R_6$ each denote methyl or $R_5$ denotes alkyl with 1-3 carbon atoms and $R_6$ denotes hydrogen.

5. Method according to claim 1, characterized in that 3-aminocarbonylmethoxy-5-phenylpyrazole compounds according to claim 1 are produced, in which

$R_1$ and $R_7$ and Z have the meaning given in claim 1 and

$R_8$ denotes hydrogen or alkyl with 1-2 carbon atoms and

$R_9$ denotes hydrogen or alkyl with 1-2 carbon atoms or

$R_8$ and $R_9$ together with the nitrogen atom to which they are linked, represent a pyrrolidine-, piperdine- or morpholine ring or
$R_8$ denotes hydrogen or methyl and
$R_9$ together with the nitrogen atom and the C-atom of the alkylene chain Z adjacent thereto represents a pyrrolidine ring.

6. Method according to claim 6, characterized in that 3-aminocarbonylmethoxy-5-phenylpyrazole compounds according to claim 1 are produced, in which
$R_1$ denotes hydrogen or alkyl with 1-2 carbon atoms,
$R_2$ denotes hydrogen or methyl,
$R_3$ denotes hydrogen, halogen, methoxy or methyl,
$R_4$ denotes hydrogen, halogen, methoxy or methyl,
$R_5$ and $R_6$ each denote methyl,
$R_7$ denotes hydrogen,
Z represents an alkylene chain with 2-4 carbon atoms or the 2-hydroxypropylene chain, and
$R_8$ denotes hydrogen or alkyl with 1-2 carbon atoms and
$R_9$ denotes alkyl with 1-2 carbon atoms or
$R_8$ and $R_9$, together with the nitrogen atom to which they are linked, form a pyrrolidine- or piperidine ring.

7. Method according to claim 6, characterized in that 3-aminocarbonylmethoxy-5-phenylpyrazole compounds according to claim 6 are produced, in which $R_1$ denotes hydrogen or methyl.

8. Method according to claim 6, characterized in that 3-aminocarbonylmethoxy-5-phenylpyrazole compounds according to claim 6 are produced, in which Z represents an alkylene chain with 3 or 4 carbon atoms or the 2-hydroxypropylene chain.

9. Method according to claim 1, characterized in that 3-aminocarbonylmethoxy-5-phenylpyrazole compounds according to claim 6 are produced, in which $R_1$ denotes an alkyl group with 1-2 carbon atoms and Z represents an ethylene chain.

10. Method according to claim 1, characterized in that 3-aminocarbonylmethoxy-5-phenylpyrazole compounds according to claim 6 are produced, in which $R_8$ and $R_9$ each denote alkyl with 1-2 carbon atoms or
$R_8$ and $R_9$ together with the nitrogen atom to which they are linked form a pyrrolidine- or piperidine ring.

11. Method according to claim 1, characterized in that as compound of the Formula I 3-[2-(3-diethylaminopropylaminocarbonyl)-propyl-2-oxy]-5-phenylpyrazole and its acid addition salts are produced.

12. Method according to claim 1, characterized in that as compound of the Formula I 3-[2-(3-diethylamino-2-hydroxypropylaminocarbonyl)-propyl-2-oxy]-5-phenyl-pyrazole and its acid addition salts are produced.

13. Method according to claim 1, characterized in that as compound of the Formula I 3-[1-(2-pyrrolidinoethylaminocarbonyl)-propyl-1-oxy]-5-(4-chlorophenyl)-pyrazole and its acid addition salts are produced.

14. Method for the production of compounds of the general Formula III

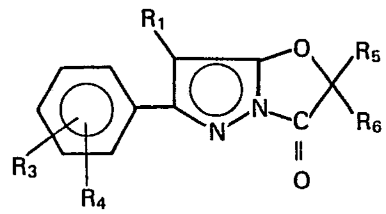

III

in which
$R_1$ denotes hydrogen or alkyl with 1-4 carbon atoms,
$R_3$ denotes hydrogen, halogen, alkyl with 1-4 carbon atoms or alkoxy with 1-4 carbon atoms and
$R_4$ denotes hydrogen, halogen alkyl with 1-4 carbon atoms, alkoxy with 1-4 carbon atoms or, if $R_3$ is hydrogen, also denotes trifluormethyl, nitro or hydroxy or
$R_3$ and $R_4$ are linked to two adjacent carbon atoms and together represent an alkylene dioxy group with 1-2 carbon atoms,
$R_5$ denotes hydrogen or alkyl with 1-4 carbon atoms and
$R_6$ denotes hydrogen or methyl or
$R_5$ and $R_6$ together form an alkylene chain with 3-5 carbon atoms,
characterized in that compounds of the general Formula II'

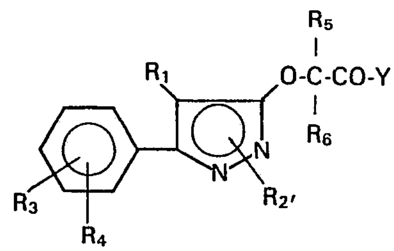

II'

in which $R_1$, $R_3$, $R_4$, $R_5$ and $R_6$ have the above meaning, $R_2'$ denotes hydrogen and Y denotes a reactive group, are condensed.

15. Method for the production of compounds of the general Formula IV

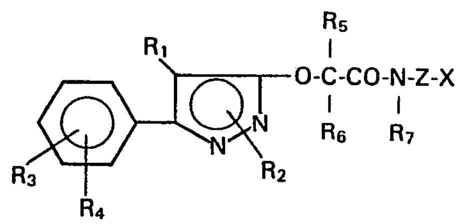

IV

in which
$R_1$ denotes hydrogen or alkyl with 1-4 carbon atoms,
$R_2$ is arranged in position 1 or 2 and denotes hydrogen or alkyl with 1-4 carbon atoms,
$R_3$ denotes hydrogen, halogen, alkyl with 1-4 carbon atoms or alkoxy with 1-4 carbon atoms and
$R_4$ denotes hydrogen, halogen alkyl with 1-4 carbon atoms, alkoxy with 1-4 carbon atoms or, if $R_3$ is hydrogen, also denotes trifluormethyl, nitro or hydroxy or

$R_3$ and $R_4$ are linked to two adjacent carbon atoms and together represent an alkylene dioxy group with 1-2 carbon atoms,
$R_5$ denotes hydrogen or alkyl with 1-4 carbon atoms and
$R_6$ denotes hydrogen or methyl or
$R_5$ and $R_6$ together form an alkylene chain with 3-5 carbon atoms,
$R_7$ denotes hydrogen or alkyl with 1-4 carbon atoms,
Z denotes an alkylene chain with 2-5 carbon atoms or denotes the 2-hydroxypropylene chain, and
X denotes an aminolytically separable group,
characterized in that compounds of the general Formula II

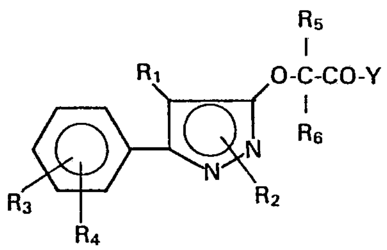

II

in which $R_1$, $R_2$, $R_3$, $R_4$, $R_5$ and $R_6$ have the above meaning and Y denotes a reactive group are reacted with compounds of the general Formula X

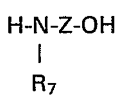

X

in which $R_7$ and Z have the above meaning, and subsequently in the obtained reaction products the final hydroxy group of the 3-side chain is converted into an aminolytically separable group X.

**Revendications pour les Etats contractants:**
BE, CH, DE. FR, GB, IT, LU, NL, SE

1. 3-aminocarbonylméthoxy-5-phénylpyrazoles, composés de formule générale I:

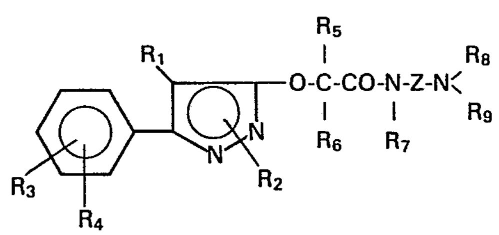

I

dans laquelle
$R_1$ représente un atome d'hydrogène ou un groupe alkyle ayant 1 à 4 atomes de carbone,
$R_2$ est placé en position 1 ou 2, et il représente un atome d'hydrogène ou un groupe alkyle ayant 1 à 4 atomes de carbone,
$R_3$ représente un atome d'hydrogène ou d'halogène, un groupe alkyle ayant 1 à 4 atomes de carbone ou un groupe alcoxy ayant 1 à 4 atomes de carbone,
$R_4$ représente un atome d'hydrogène ou d'halogène, un groupe alkyle ayant 1 à 4 atomes de carbone, un groupe alcoxy ayant 1 à 4 atomes de carbone ou, si $R_3$ représente un atome d'hydrogène, $R_4$ peut représenter aussi un groupe trifluorométhyle, nitro ou hydroxy, ou bien
$R_3$ et $R_4$ sont fixés sur deux atomes voisins de carbone et ils représentent ensemble un groupe alkylènedioxy ayant 1 ou 2 atomes de carbone,
$R_5$ représente un atome d'hydrogène ou un groupe alkyle ayant 1 à 4 atomes de carbone, et
$R_6$ représente un atome d'hydrogène ou un groupe méthyle, ou bien
$R_5$ et $R_6$ forment ensemble une chaîne alkylène ayant 3 à 5 atomes de carbone,
$R_7$ représente un atome d'hydrogène ou un groupe alkyle ayant 1 à 4 atomes de carbone,
Z représente une chaîne alkylène ayant 2 à 5 atomes de carbone ou bien la chaîne 2-hydroxypropylène,
$R_8$ représente un atome d'hydrogène ou un groupe alkyle ayant 1 à 4 atomes de carbone, et
$R_9$ représente un atome d'hydrogène ou un groupe alkyle ayant 1 à 4 atomes de carbone, ou bien
$R_8$ et $R_9$, pris avec l'atome d'azote auquel ils sont fixés, représentent un groupe hétérocyclique de formule générale a:

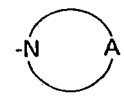

a

dans laquelle A représente une chaîne alkylène ayant 4 à 5 atomes de carbone et éventuellement substituée par 1 à 2 groupes méthyle, ou bien la chaîne $C_2H_4$-O-$C_2H_4$, ou bien, si Z est une chaîne alkylène,
$R_8$ peut aussi représenter un atome d'hydrogène ou un groupe alkyle ayant 1 à 4 atomes de carbone, et
$R_9$ est une chaîne alkylène et, avec l'atome d'azote auquel cette chaîne est fixée, et avec l'atome de carbone, voisin de cet atome d'azote, de la chaîne alkylène Z, il forme un noyau pyrrolidine ou pipéridine,
ainsi que leurs sels d'addition d'acides.

2. 3-aminocarbonylméthoxy-5-phénylpyrazoles selon la revendication 1, dans lesquels:
$R_3$ à $R_9$ et Z ont le sens indiqué à la revendication 1,
$R_1$ représente un atome d'hydrogène ou un groupe alkyle ayant 1 ou 2 atomes de carbone, et
$R_2$ représente un atome d'hydrogène ou un groupe méthyle.

3. 3-aminocarbonyméthoxy-5-phénylpyrazoles selon la revendication 1, dans lesquels:
$R_1$ à $R_4$, $R_7$ à $R_9$ et Z ont le sens indiqué à la revendication 1, et
$R_5$ et $R_6$ comportent ensemble 3 atomes de carbone.

4. 3-aminocarbonylméthoxy-5-phénylpyrazoles selon la revendication 3, dans lesquels:
$R_5$ et $R_6$ représentent chacun un groupe méthyle, ou bien

$R_5$ représente un groupe alkyle ayant 1 à 3 atomes de carbone et

$R_6$ représente un atome d'hydrogène.

5. 3-aminocarbonylméthoxy-5-phénylpyrazoles selon la revendication 1, dans lesquels:

$R_1$ à $R_7$ et Z ont le sens indiqué à la revendication 1, et

$R_8$ représente un atome d'hydrogène ou un groupe alkyle ayant 1 à 2 atomes de carbone, et

$R_9$ représente un atome d'hydrogène ou un groupe alkyle ayant 1 à 2 atomes de carbone, ou bien

$R_8$ et $R_9$, pris avec l'atome d'azote auquel ils sont reliés, représentent un noyau pyrrolidine, pipéridine ou morpholine, ou

$R_8$ représente un atome d'hydrogène ou un groupe méthyle, et

$R_9$, pris avec l'atome d'azote et l'atome de carbone, voisin de l'azote de la chaîne alkylène Z, représentent un noyau pyrrolidine.

6. 3-aminocarbonylméthoxy-5-phénylpyrazoles selon la revendication 1, dans lesquels:

$R_1$ représente un atome d'hydrogène ou un groupe alkyle ayant 1 ou 2 atomes de carbone,

$R_2$ représente un atome d'hydrogène ou un groupe méthyle,

$R_3$ représente un atome d'hydrogène, un atome d'halogène, un groupe méthoxy ou un groupe méthyle,

$R_4$ représente un atome d'hydrogène, un atome d'halogène, un groupe méthoxy ou un groupe méthyle,

$R_5$ et $R_6$ représentent chacun un groupe méthyle,

$R_7$ représente un atome d'hydrogène,

Z représente une chaîne alkylène ayant 2 à 4 atomes de carbone ou la chaîne 2-hydroxypropylène, et

$R_8$ représente un atome d'hydrogène ou un groupe alkyle ayant 1 ou 2 atomes de carbone, et

$R_9$ représente un groupe alkyle ayant 1 ou 2 atomes de carbone, ou bien

$R_8$ et $R_9$, pris avec l'atome d'azote auquel ils sont reliés, forment un noyau pyrrolidine ou pipéridine.

7. 3-aminocarbonylméthoxy-5-phénylpyrazoles selon la revendication 6, dans lesquels $R_1$ représente un atome d'hydrogène ou un groupe méthyle.

8. 3-aminocarbonylméthoxy-5-phénylpyrazoles selon la revendication 6, dans lesquels Z représente une chaîne alkylène ayant 3 ou 4 atomes de carbone ou la chaîne 2-hydroxypropylène.

9. 3-aminocarbonylméthoxy-5-phénylpyrazoles selon la revendication 6, dans lesquels $R_1$ représente un groupe alkyle ayant 1 ou 2 atomes de carbone et Z est une chaîne éthylène.

10. 3-aminocarbonylméthoxy-5-phénylpyrazoles selon la revendication 6, dans lesquels $R_8$ et $R_9$ représentent chacun un groupe alkyle ayant 1 ou 2 atomes de carbone, ou bien

$R_8$ et $R_9$, pris avec l'atome d'azote auquel ils sont reliés, forment un noyau pyrrolidine ou pipéridine.

11. 3-[2-(3-diéthylaminopropylaminocarbonyl)-propyl-2-oxy]-5-phénylpyrazole selon la revendication 1, et ses sels d'addition d'acides.

12. 3-[2-(3-diéthylamino-2-hydroxypropylaminocarbonyl)-propyl-2-oxy]-5-phénylpyrazole selon la revendication 1 et ses sels d'addition d'acides.

13. 3-[1-(2-pyrrolidinoéthylaminocarbonyl)-propyl-1-oxy]-5-(4-chlorphényl)-pyrazole selon la revendication 1 et ses sels d'addition d'acides.

14. Médicament contenant une quantité pharmacologiquement efficace d'un 3-aminocarbonylméthoxy-5-phénylpyrazole selon la revendication 1, et des adjuvants et/ou excipients ou supports usuels.

15. Procédé pour préparer des composés de formule générale I selon la revendication I:

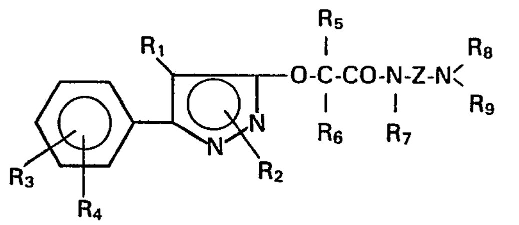

I

dans laquelle $R_1$, $R_2$, $R_3$, $R_4$, $R_5$, $R_6$, $R_7$, $R_8$, $R_9$ et Z ont le sens indiqué à la revendication 1,
ainsi que leurs sels d'addition d'acides, caractérisé en ce que:

a) on fait réagir des composés de formule générale II ou III:

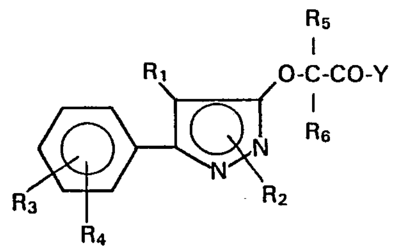

II

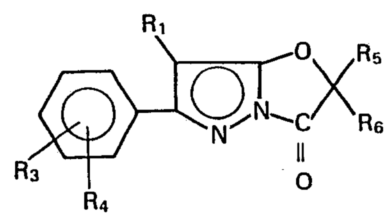

III

dans lesquelles $R_1$, $R_2$, $R_3$, $R_4$, $R_5$ et $R_6$ ont le sens ci-dessus et Y est un groupe réactif, avec des composés de formule générale VIII:

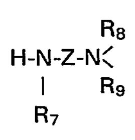

VIII

dans laquelle $R_7$, Z, $R_8$ et $R_9$ ont les sens ci-dessus, ou

b) on fait réagir des composés de formule générale IV:

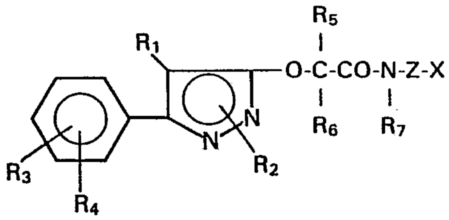

IV

dans laquelle $R_1$, $R_2$, $R_3$, $R_4$, $R_5$, $R_6$, $R_7$ et Z ont le sens ci-dessus et X représente un groupe pouvant être séparé par aminolyse, avec des composés de formule générale V:

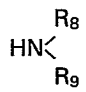

V

dans laquelle $R_8$ et $R_9$ ont le sens ci-dessus, ou bien

c) on fait réagir des composés de formule générale VI:

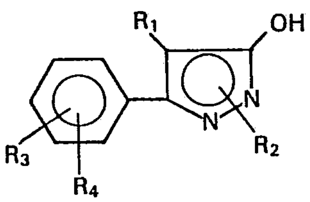

VI

dans laquelle $R_1$, $R_2$, $R_3$ et $R_4$ ont le sens ci-dessus et/ou les 5-phénylpyrazoline-3-ones tautomères, correspondantes avec des composés de formule générale VII:

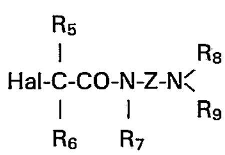

VII

dans laquelle $R_5$, $R_6$, $R_7$, Z, $R_8$ et $R_9$ ont le sens ci-dessus et Hal représente un halogène, ou

d) pour préparer des composés de formule générale Ia:

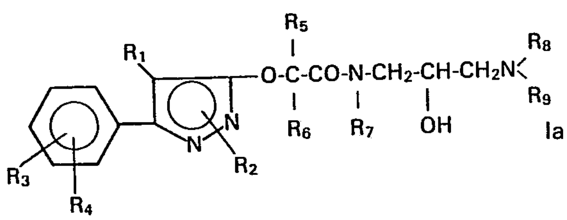

Ia

dans laquelle $R_1$, $R_2$, $R_3$, $R_4$, $R_5$, $R_6$, $R_7$, $R_8$ et $R_9$ ont le sens ci-dessus, on fait réagir des composés de formule générale IX:

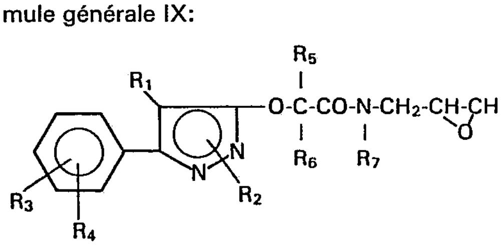

IX

dans laquelle $R_1$, $R_2$, $R_3$, $R_4$, $R_5$, $R_6$ et $R_7$ ont le sens ci-dessus, avec un composé de formule générale V, et éventuellement, dans des composés de formule générale I dans laquelle $R_4$ représente un groupe méthoxy, on scinde le groupe méthoxy pour obtenir un groupe hydroxy,

et éventuellement on transforme des composés libres de formule I en leurs sels d'addition d'acides ou bien on transforme les sels d'addition d'acides en les composés libres de formule I.

16. Composés de formule générale III:

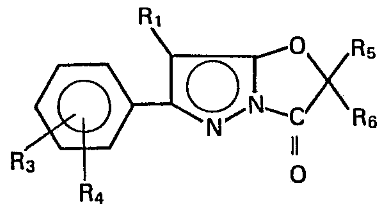

III

dans laquelle

$R_1$ représente un atome d'hydrogène ou un groupe alkyle ayant 1 à 4 atomes de carbone,

$R_3$ représente un atome d'hydrogène ou d'halogène, un groupe alkyle ayant 1 à 4 atomes de carbone ou un groupe alcoxy ayant 1 à 4 atomes de carbone, et

$R_4$ représente un atome d'hydrogène ou d'halogène, un groupe alkyle ayant 1 à 4 atomes de carbone, alcoxy ayant 1 à 4 atomes de carbone ou, si $R_3$ est un atome d'hydrogène, $R_4$ représente aussi un groupe trifluorométhyle, nitro ou hydroxy, ou bien

$R_3$ et $R_4$ sont fixés sur deux atomes voisins de carbone et constituent ensemble un groupe alkylènedioxy ayant 1 ou 2 atomes de carbone,

$R_5$ représente un atome d'hydrogène ou un groupe alkyle ayant 1 à 4 atomes de carbone, et

$R_6$ représente un atome d'hydrogène ou un groupe méthyle, ou bien

$R_5$ et $R_6$ forment ensemble une chaîne alkylène ayant 3 à 5 atomes de carbone.

17. Composé de formule générale IV:

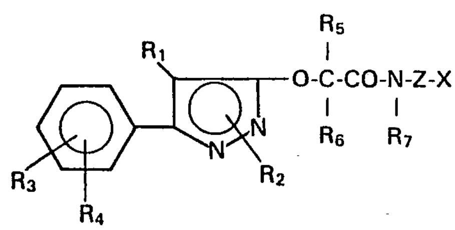

IV

dans laquelle

$R_1$ représente un atome d'hydrogène ou un groupe alkyle ayant 1 à 4 atomes de carbone,

$R_2$ est fixé en position 1 ou 2 et il représente un atome d'hydrogène ou un groupe alkyle ayant 1 à 4 atomes de carbone,

$R_3$ représente un atome d'hydrogène ou d'halogène, un groupe alkyle ayant 1 à 4 atomes de carbone ou alcoxy ayant 1 à 4 atomes de carbone, et

$R_4$ représente un atome d'hydrogène ou d'halogène ou un groupe alkyle ayant 1 à 4 atomes de carbone, un groupe alcoxy ayant 1 à 4 atomes de carbone ou bien, si $R_3$ est un atome d'hydrogène, $R_4$ représente aussi un groupe trifluorométhyle, nitro ou hydroxy, ou bien

$R_3$ et $R_4$ sont fixés sur deux atomes voisins de carbone et ils constituent ensemble un groupe alkylènedioxy ayant 1 ou 2 atomes de carbone,

$R_5$ représente un atome d'hydrogène ou un groupe alkyle ayant 1 à 4 atomes de carbone, et

$R_6$ représente un atome d'hydrogène ou un groupe méthyle, ou

$R_5$ et $R_6$ forment ensemble une chaîne alkylène ayant 3 à 5 atomes de carbone,

$R_7$ représente un atome d'hydrogène ou un groupe alkyle ayant 1 à 4 atomes de carbone,

Z représente une chaîne alkylène ayant 2 à 5 atomes de carbone ou la chaîne 2-hydroxypropylène, et

X représente un groupe pouvant être séparé par aminolyse.

**Revendications pour l'Etat contractant:** AT

1. Procédé pour préparer des composés de formule générale I:

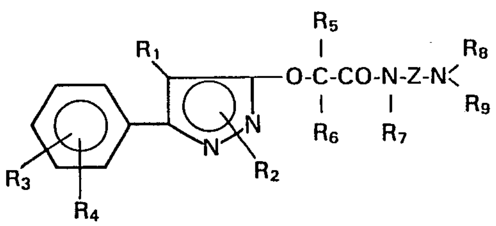

I

dans laquelle

$R_1$ représente un atome d'hydrogène ou un groupe alkyle ayant 1 à 4 atomes de carbone,

$R_2$ est placé en position 1 ou 2, et il représente un atome d'hydrogène ou un groupe alkyle ayant 1 à 4 atomes de carbone,

$R_3$ représente un atome d'hydrogène ou d'halogène, un groupe alkyle ayant 1 à 4 atomes de carbone ou un groupe alcoxy ayant 1 à 4 atomes de carbone,

$R_4$ représente un atome d'hydrogène ou d'halogène, un groupe alkyle ayant 1 à 4 atomes de carbone, un groupe alcoxy ayant 1 à 4 atomes de carbone ou, si $R_3$ représente un atome d'hydrogène, $R_4$ peut représenter aussi un groupe trifluorométhyle, nitro ou hydroxy, ou bien

$R_3$ et $R_4$ sont fixés sur deux atomes voisins de carbone et ils représentent ensemble un groupe alkylènedioxy ayant 1 ou 2 atomes de carbone,

$R_5$ représente un atome d'hydrogène ou un groupe alkyle ayant 1 à 4 atomes de carbone, et

$R_6$ représente un atome d'hydrogène ou un groupe méthyle, ou bien

$R_5$ et $R_6$ forment ensemble une chaîne alkylène ayant 3 à 5 atomes de carbone,

$R_7$ représente un atome d'hydrogène ou un groupe alkyle ayant 1 à 4 atomes de carbone,

Z représente une chaîne alkylène ayant 2 à 5 atomes de carbone ou bien la chaîne 2-hydroxypropylène,

$R_8$ représente un atome d'hydrogène ou un groupe alkyle ayant 1 à 4 atomes de carbone, et

$R_9$ représente un atome d'hydrogène ou un groupe alkyle ayant 1 à 4 atomes de carbone, ou bien

$R_8$ et $R_9$, pris avec l'atome d'azote auquel ils sont fixés, représentent un groupe hétérocyclique de formule générale a:

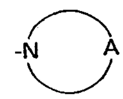

a

dans laquelle A représente une chaîne alkylène ayant 4 à 5 atomes de carbone et éventuellement substituée par 1 à 2 groupes méthyle, ou bien la chaîne $C_2H_4$-O-$C_2H_4$, ou bien, si Z est une chaîne alkylène,

$R_8$ peut aussi représenter un atome d'hydrogène ou un groupe alkyle ayant 1 à 4 atomes de carbone, et

$R_9$ est une chaîne alkylène et, avec l'atome d'azote auquel cette chaîne est fixée, et avec l'atome de carbone, voisin de cet atome d'azote, de la chaîne alkylène Z, il forme un noyau pyrrolidine ou pipéridine,

ainsi que leurs sels d'addition d'acides, caractérisé en ce que:

a) on fait réagir des composés de formule générale II ou III:

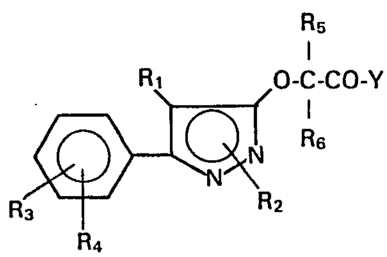

II

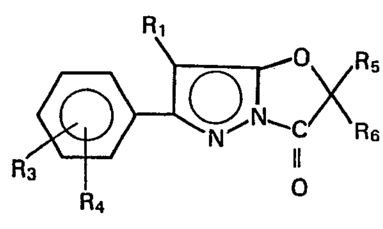

III

dans lesquelles $R_1$, $R_2$, $R_3$, $R_4$, $R_5$ et $R_6$ ont le sens ci-dessus et Y est un groupe réactif, avec des composés de formule générale VIII:

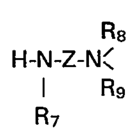

VIII

dans laquelle $R_7$, Z, $R_8$ et $R_9$ ont les sens ci-dessus, ou

b) on fait réagir des composés de formule générale IV:

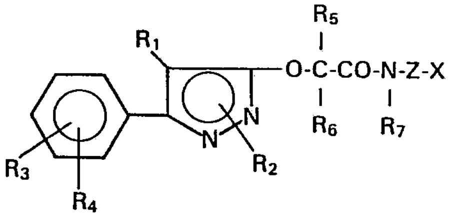

IV

dans laquelle $R_1$, $R_2$, $R_3$, $R_4$, $R_5$, $R_6$, $R_7$ et Z ont le sens ci-dessus et X représente un groupe pouvant être séparé par aminolyse, avec des composés de formule générale V:

$$HN\begin{matrix} R_8 \\ R_9 \end{matrix} \qquad V$$

dans laquelle $R_8$ et $R_9$ ont le sens ci-dessus, ou bien

c) on fait réagir des composés de formule générale VI:

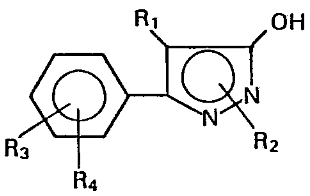

VI

dans laquelle $R_1$, $R_2$, $R_3$ et $R_4$ ont le sens ci-dessus et/ou les 5-phénylpyrazoline-3-ones tautomères, correspondantes avec des composés de formule générale VII:

$$\underset{\underset{R_6}{|}}{\overset{\overset{R_5}{|}}{Hal\text{-}C}}\text{-}CO\text{-}\underset{\underset{R_7}{|}}{N}\text{-}Z\text{-}N\begin{matrix} R_8 \\ R_9 \end{matrix} \qquad VII$$

dans laquelle $R_5$, $R_6$, $R_7$, Z, $R_8$ et $R_9$ ont le sens ci-dessus et Hal représente un halogène, ou

d) pour préparer des composés de formule générale Ia:

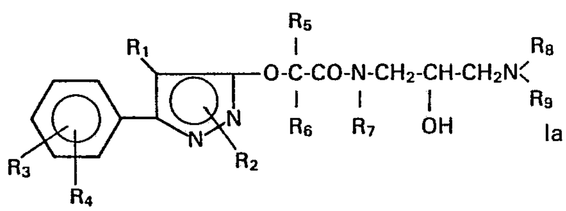

Ia

dans laquelle $R_1$, $R_2$, $R_3$, $R_4$, $R_5$, $R_6$, $R_7$, $R_8$ et $R_9$ ont le sens ci-dessus, on fait réagir des composés de formule générale IX:

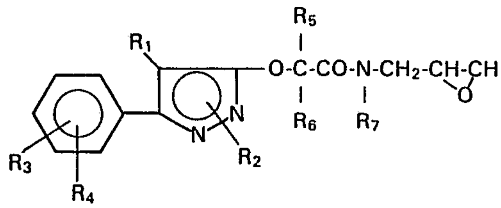

IX

dans laquelle $R_1$, $R_2$, $R_3$, $R_4$, $R_5$, $R_6$ et $R_7$ ont le sens ci-dessus, avec un composé de formule générale V, et éventuellement, dans des composés de formule générale I dans laquelle $R_4$ représente un groupe méthoxy, on scinde le groupe méthoxy pour obtenir un groupe hydroxy,

et éventuellement on transforme des composés libres de formule I en leurs sels d'addition d'acides ou bien on transforme les sels d'addition d'acides en composés libres de formule I.

2. Procédé selon la revendication 1, caractérisé en ce qu'on prépare des 3-aminocarbonylméthoxy-5-phénylpyrazoles selon la revendication 1, dans lesquels:

$R_3$ à $R_9$ et Z ont les sens indiqué à la revendication 1,

$R_1$ représente un atome d'hydrogène ou un groupe alkyle ayant 1 ou 2 atomes de carbone, et

$R_2$ représente un atome d'hydrogène ou un groupe méthyle.

3. Procédé selon la revendication 1, caractérisé en ce qu'on prépare des 3-aminocarbonylméthoxy-5-phénylpyrazoles selon la revendication 1, dans lesquels:

$R_1$ à $R_4$, $R_7$ à $R_9$ et Z ont le sens indiqué à la revendication 1, et

$R_5$ et $R_6$ possèdent ensemble 3 atomes de carbone.

4. Procédé selon la revendication 1, caractérisé en ce qu'on prépare des 3-aminocarbonylméthoxy-5-phénylpyrazoles selon la revendication 3, dans lesquels:

$R_5$ et $R_6$ représentent chacun un groupe méthyle, ou bien

$R_5$ représente un groupe alkyle ayant 1 à 3 atomes de carbone et

$R_6$ représente un atome d'hydrogène.

5. Procédé selon la revendication 1, caractérisé en ce qu'on prépare des 3-aminocarbonylméthoxy-5-phénylpyrazoles selon la revendication 1, dans lesquels:

$R_1$ à $R_7$ et Z ont le sens indiqué à la revendication 1, et

$R_8$ représente un atome d'hydrogène ou un groupe alkyle ayant 1 à 2 atomes de carbone, et

$R_9$ représente un atome d'hydrogène ou un groupe alkyle ayant 1 à 2 atomes de carbone, ou bien

$R_8$ et $R_9$, pris avec l'atome d'azote auquel ils sont reliés, constituent un noyau pyrrolidine, pipéridine ou morpholine, ou

$R_8$ représente un atome d'hydrogène ou un groupe méthyle, et

$R_9$, pris avec l'atome d'azote et l'atome de carbone, voisin de celui-ci de la chaîne alkylène Z, représente un noyau pyrrolidine.

6. Procédé selon la revendication 1, caractérisé

en ce qu'on prépare des 3-aminocarbonylméthoxy-5-phénylpyrazoles selon la revendication 1, dans lesquels:

$R_1$ représente un atome d'hydrogène ou un groupe alkyle ayant 1 ou 2 atomes de carbone,

$R_2$ représente un atome d'hydrogène ou un groupe méthyle,

$R_3$ représente un atome d'hydrogène, ou d'halogène, ou un groupe méthoxy ou méthyle,

$R_4$ représente un atome d'hydrogène, ou d'halogène, ou un groupe méthoy ou méthyle,

$R_5$ et $R_6$ représentent chacun un groupe méthyle,

$R_7$ représente un atome d'hydrogène,

Z constitue une chaîne alkylène ayant 2 à 4 atomes de carbone ou est la chaîne 2-hydroxypropylène, et

$R_8$ représente un atome d'hydrogène ou un groupe alkyle ayant 1 ou 2 atomes de carbone, et

$R_9$ représente un groupe alkyle ayant 1 ou 2 atomes de carbone, ou bien

$R_8$ et $R_9$, pris avec l'atome d'azote auquel ils sont reliés, forment un noyau pyrrolidine ou pipéridine.

7. Procédé selon la revendication 6, caractérisé en ce qu'on prépare des 3-aminocarbonylméthoxy-5-phénylpyrazoles selon la revendication 6, dans lesquels $R_1$ représente un atome d'hydrogène ou un groupe méthyle.

8. Procédé selon la revendication 6, caractérisé en ce qu'on prépare des 3-aminocarbonylméthoxy-5-phénylpyrazoles selon la revendication 6, dans lesquels Z représente une chaîne alkylène ayant 3 ou 4 atomes de carbone ou la chaîne 2-hydroxypropylène.

9. Procédé selon la revendication 6, caractérisé en ce qu'on prépare des 3-aminocarbonylméthoxy-5-phénylpyrazoles selon la revendication 6, dans lesquels $R_1$ représente un groupe alkyle ayant 1 ou 2 atomes de carbone et Z est une chaîne éthylène.

10. Procédé selon la revendication 6, caractérisé en ce qu'on prépare des 3-aminocarbonylméthoxy-5-phénylpyrazoles selon la revendication 6, dans lesquels $R_8$ et $R_9$ représentent chacun un groupe alkyle ayant 1 ou 2 atomes de carbone, ou bien

$R_8$ et $R_9$, pris avec l'atome d'azote auquel ils sont reliés, forment un noyau pyrrolidine ou pipéridine.

11. Procédé selon la revendication 1, caractérisé en ce qu'on prépare, comme composé de formule I, le 3-[2-(3-diéthylaminopropylaminocarbonyl)-propyl-2-oxy]-5-phénylpyrazole et ses sels d'addition d'acides.

12. Procédé selon la revendication 1, caractérisé en ce qu'on prépare, comme composé de formule I, le 3-[2-(3-diéthylamino-2-hydroxypropylaminocarbonyl)-propyl-2-oxy]-5-phénylpyrazole et ses sels d'addition d'acides.

13. Procédé selon la revendication 1, caractérisé en ce qu'on prépare, comme composé de formule I, le 3-[1-(2-pyrrolidinoéthylaminocarbonyl)-propyl-1-oxy]-5-(4-chlorphényl)-pyrazole et ses sels d'addition d'acides.

14. Procédé pour préparer des composés de formule générale III:

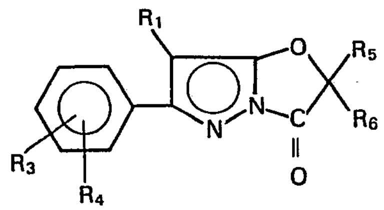

III

dans laquelle

$R_1$ représente un atome d'hydrogène ou un groupe alkyle ayant 1 à 4 atomes de carbone,

$R_3$ représente un atome d'hydrogène ou d'halogène, un groupe alkyle ayant 1 à 4 atomes de carbone ou un groupe alcoxy ayant 1 à 4 atomes de carbone, et

$R_4$ représente un atome d'hydrogène ou d'halogène, un groupe alkyle ayant 1 à 4 atomes de carbone, alcoxy ayant 1 à 4 atomes de carbone ou, si $R_3$ est un atome d'hydrogène, $R_4$ représente aussi un groupe trifluorométhyle, nitro ou hydroxy, ou bien

$R_3$ et $R_4$ sont fixés sur deux atomes voisins de carbone et constituent ensemble un groupe alkylènedioxy ayant 1 ou 2 atomes de carbone,

$R_5$ représente un atome d'hydrogène ou un groupe alkyle ayant 1 à 4 atomes de carbone, et

$R_6$ représente un atome d'hydrogène ou un groupe méthyle, ou bien

$R_5$ et $R_6$ forment ensemble une chaîne alkylène ayant 3 à 5 atomes de carbone,

caractérisé en ce qu'on condense des composés de formule générale II' :

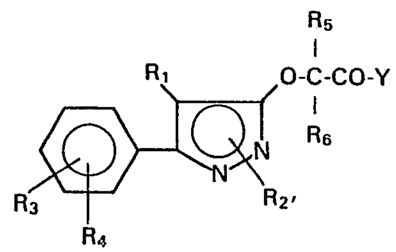

II'

dans laquelle $R_1$, $R_3$, $R_4$, $R_5$ et $R_6$ ont le sens ci-dessu, $R_2'$ représente un atome d'hydrogène et Y représente un groupe réactif.

15. Procédé pour préparer des composés de formule générale IV:

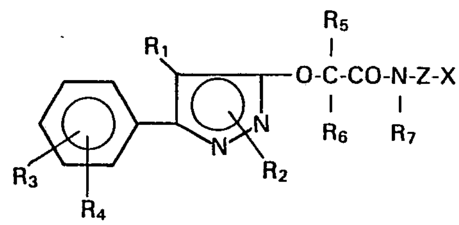

IV

dans laquelle

$R_1$ représente un atome d'hydrogène ou un groupe alkyle ayant 1 à 4 atomes de carbone,

$R_2$ est fixé en position 1 ou 2 et il représente un atome h'hydrogène ou un groupe alkyle ayant 1 à 4 atomes de carbone,

$R_3$ représente un atome d'hydrogène ou d'halogène, un groupe alkyle ayant 1 à 4 atomes de carbone ou alcoxy ayant 1 à 4 atomes de carbone, et

$R_4$ représente un atome d'hydrogène ou d'halogène ou un groupe alkyle ayant 1 à 4 atomes de carbone, un groupe alcoxy ayant 1 à 4 atomes de carbone ou bien, si $R_3$ est un atome d'hydrogène, $R_4$ représente aussi un groupe trifluorométhyle, nitro ou hydroxy, ou bien

$R_3$ et $R_4$ sont fixés sur deux atomes voisins de carbone et ils constituent ensemble un groupe alkylènedioxy ayant 1 ou 2 atomes de carbone,

$R_5$ représente un atome d'hydrogène ou un groupe alkyle ayant 1 à 4 atomes de carbone, et

$R_6$ représente un atome d'hydrogène ou un groupe méthyle, ou

$R_5$ et $R_6$ forment ensemble une chaîne alkylène ayant 3 à 5 atomes de carbone,

$R_7$ représente un atome d'hydrogène ou un groupe alkyle ayant 1 à 4 atomes de carbone,

Z représente une chaîne alkylène ayant 2 à 5 atomes de carbone ou la chaîne 2-hydroxypropylène, et

X représente un groupe pouvant être séparé par aminolyse,

caractérisé en ce qu'on fait réagir des composés de formule générale II:

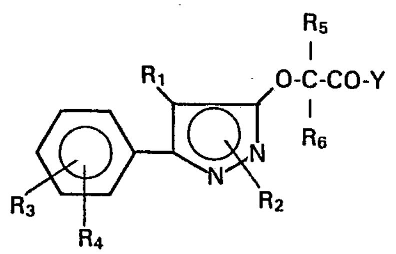

II

dans laquelle $R_1$, $R_2$, $R_3$, $R_4$, $R_5$ et $R_6$ ont le sens ci-dessus et Y représente un groupe réactif, avec des composés de formule générale X:

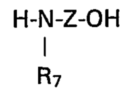

X

dans laquelle $R_7$ et Z ont le sens ci-dessus, puis, dans les produits de réaction obtenus, on transforme le groupe hydroxy terminal de la chaîne latérale 3 en un groupe X scindable par aminolyse.